# DEMANDE DE BREVET EUROPEEN

(11) **EP 4 049 654 A1**
(43) Date de publication de la demande: **31.08.2022**
(21) Numéro de dépôt: 22158783.5
(22) Date de dépôt: 25.02.2022
(51) Int. Cl.: A61K 9/16, A61K 31/05

(54) **COMPOSITION COMPRENANT AU MOINS UN CANNABINOÏDE NON-EUPHORISANT NATUREL OU SYNTHÉTIQUE ET SON PROCÉDÉ DE FABRICATION**

(30) Priorité: 26.02.2021 BE 202105139
(71) Demandeur: Eleonor, 1410 Waterloo (BE); Tilman, 5377 Baillonville (BE)
(72) Inventeur: LOIRA-PASTORIZA, Cristina, 1120 Bruxelles (BE); PRIAN, Mayéli, 6987 Rendeux (BE); PRIEM, Fabian, 1410 Waterloo (BE); DIERCKXSENS, Yvan, 3090 Overijse (BE)

(57) **Abrégé**

La présente invention porte sur une composition sous forme d'un extrudat thermoformé friable / cassant comprenant au moins un cannabinoïde non-euphorisant naturel ou synthétique et au moins un support naturel ou synthétique choisi dans le groupe constitué des acides aminés, des peptides et des polypeptides naturels ou synthétiques, des protéines naturelles ou synthétiques, des saccharides naturels ou synthétiques, des lipides naturels ou synthétiques, de l'urée, de leurs dérivés et de leurs mélanges.

## Description

La présente invention se rapporte à une composition comprenant au moins un cannabinoïde non-euphorisant naturel ou synthétique, à son procédé de fabrication et à son utilisation.

Selon l'invention, un cannabinoïde est une molécule dont la structure chimique de base est la suivante : 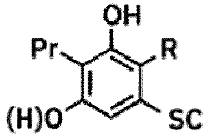 dans laquelle, Pr = groupement prényl ; R = H ou COOH ; SC (side-chain) = chaine latérale isoprényl en position para ou chaine latérale alkyl en position para ou chaine latérale aralkyl en position para.

Cette structure chimique est présentée dans le document de Gülck et al. (Trends in Plant Science, 2020, Vol. 25, No. 10) qui indique que le terme (phyto-)cannabinnoïde définit les méroterpénoïdes avec un noyau résorcinyl présentant une chaine latérale isoprényl, alkyl ou aralkyl en position para.

Par les termes « cannabinoïde non-euphorisant », il est entendu, au sens de la présente invention, un cannabinoïde qui, chez un individu/un animal, une fois absorbé/consommé, n'altère pas majoritairement et significativement les facultés mentales et/ou motrices de l'individu/l'animal compatibles avec les activités de la vie courante, par exemple en donnant lieu à de l'euphorie, à de l'exaltation, à des hallucinations, à de la confusion ou encore à tout autre état affectant les activités de la vie courante. A titre d'exemple, des substances provoquant de tels états et étant donc euphorisantes sont le Δ-9-tétrahydrocannabinol (THC), la cocaïne, l'ectasy ou encore l'héroïne.

Au sens de la présente invention, le terme « cannabinoïde non-euphorisant naturel ou synthétique » désigne tant les cannabinoïdes non-euphorisants d'origine naturelle et/ou végétale que les cannabinoïdes non-euphorisants de synthèse, mais aussi tous les dérivés de cannabinoïdes non-euphorisants.

Selon l'invention, ledit cannabinoïde non-euphorisant peut être notamment issu des végétaux suivants : Amorpha fruticosa, Cannabis sativa, Cylindrocarpon olidum, Glycyrrhiza acanthocarpa, Helichrysum umbraculigerum, Machaerium multiflorum, Radula spp. (Radula marginata, Radula perrottettii, Radula complanata, ...), Rhododendron spp (Rhododendron dauricum, Rhododendron anthopogonoides, ...). Cette liste est non exhaustive.

Il est reconnu que les cannabinoïdes non-euphorisants naturels ou synthétiques sont des composés très peu voire totalement insolubles dans l'eau dans laquelle ils ne se dispersent d'ailleurs que faiblement ou pas du tout, ces composés présentant dès lors de très faibles biodisponibilités.

Pourtant, malgré leur faible biodisponibilité/bioaccessibilité (c'est-à-dire malgré la faible fraction de la dose administrée qui atteint effectivement la circulation sanguine sous forme inchangée) mais aussi malgré leur faible solubilité et/ou malgré leur faible dispersion notamment dans le milieu intestinal, les effets positifs des cannabinoïdes non-euphorisants naturels ou synthétiques sur diverses pathologies en font des molécules d'intérêt en vue d'une administration à l'être humain et/ou pour un usage vétérinaire.

Malheureusement, il apparait que les formulations/compositions actuelles comprenant des cannabinoïdes non-euphorisants naturels ou synthétiques sont peu appropriées en raison de leur trop faible voire de leur non-solubilité et/ou en raison de leur trop faible voire de leur non dispersion en phase aqueuse et/ou en raison du faible relargage de ces composés au départ de ces formulations/compositions, ce qui se traduit finalement par une faible biodisponibilité/bioaccessibilité de ces molécules d'intérêt. Notons qu'il apparait aussi que les procédés actuels de fabrication de ces formulations sont contraignants et difficiles à mettre en œuvre.

Par les termes « dispersion en phase aqueuse (en milieu aqueux) », il est entendu, au sens de la présente invention, un système composé de deux phases dans lequel l'une des deux phases, appelée phase dispersée, est finement divisée dans l'autre, appelée phase dispersante. Cette dispersion peut être moléculaire (solution), colloïdale (dispersion de particules submicroniques) ou plus grossière (dispersion de particules supérieures au µm). Plus particulièrement, selon l'invention, les termes « dispersion en phase aqueuse » désignent les suspensions, constituées d'une phase solide dispersée dans une phase aqueuse (liquide).

Au sens de la présente invention, le terme « solubilité », désigne la capacité d'une substance, appelée soluté, à se dissoudre dans une autre substance, appelée solvant, pour former un mélange homogène appelé solution.

L'invention a pour but de pallier les inconvénients de l'état de la technique en procurant (1) une composition comprenant au moins un cannabinoïde non-euphorisant naturel ou synthétique dont la solubilité et/ou la dispersion en phase aqueuse (milieu aqueux) est augmentée de telle sorte que la biodisponibilité de ces composés soit significativement augmentée et (2) un procédé de fabrication d'une telle composition qui soit facile à mettre en œuvre, qui soit flexible, qui soit économiquement rentable et qui assure que ledit au moins un cannabinoïde non-euphorisant naturel ou synthétique soit présent et réparti de façon homogène dans la composition finale obtenue.

Par ailleurs, l'invention entend procurer une composition qui est stable au cours du temps, c'est-à-dire qui conserve ses propriétés en termes de solubilité et/ou de dispersion dudit au moins un cannabinoïde non-euphorisant naturel ou synthétique et qui conserve ses propriétés en termes de taux de relargage de ce composé au cours du temps au départ d'une composition (formulation) selon l'invention, ceci en particulier en phase aqueuse et plus particulièrement dans le milieu intestinal.

Pour résoudre au moins partiellement ces problèmes, il est prévu suivant l'invention, une composition sous forme d'un extrudat thermoformé friable / cassant, éventuellement conditionné en pellet, en flake, en granulé, en poudre, en comprimé effervescent ou non, en solution injectable ou non, en suspension, en gel, en pommade ou encore sous toute autre forme adéquate permettant une administration à un animal ou à un être humain, comprenant au moins un cannabinoïde non-euphorisant naturel ou synthétique et au moins un support naturel ou synthétique choisi dans le groupe constitué des acides aminés, des peptides et des polypeptides naturels ou synthétiques, des protéines naturelles ou synthétiques sélectionnées parmi les glycoprotéines, les collagènes, les hydrolysats de collagènes, les gélatines, les protéines de pois, les protéines de soja, les protéines de blé, les protéines de citrouille, les protéines de noix, les protéines de riz, les protéines de noisette, les protéines de lait, les protéines de petit pois, les protéines de lentilles, les protéines d'amandes, les protéines de pistaches, les protéines de chia, les protéines de chanvre, les protéines de lin, les protéines de courge, les protéines de sésame, les protéines de pavot, les protéines de lupin, les protéines de tournesol, les protéines d'avoine, les protéines de millet, les protéines d'orge, les protéines de seigle, leurs dérivés et leurs mélanges, des saccharides naturels ou synthétiques sélectionnés parmi l'alginate de calcium, l'alginate de sodium, l'alginate d'ammonium, l'alginate de propylène glycol, les amidons, les amidons prégélatinisés, les amidons modifiés, les fibres solubles ou insolubles, les carraghénanes, la pectine, l'hydroxypropylcellulose (HPC), le glycogène, le β-glucane, l'amylopectine, l'amylose, les dextrines, les cyclodextrines, la maltodextrine, l'isomaltose, l'isomalt, le xylane, le pullulan, l'agar-agar, les mannanes, le fucoïdane, la gomme de xanthane, la gomme de guar, la gomme mastic, la gomme arabique, la gomme d'acacia, la gomme adragante, la gomme ghatti, la gomme de karaya, la gomme de Konjac, la gomme de fenugrec, la gomme d'acajou ou anacarde, la gomme tara, la gomme de caroube, la gomme de cassia, la gomme de benjoin, la gomme cassia, la gomme gaïac, le chitosan, la chitine, la lévane, la néoserine, l'acide hyaluronique, les hyaluronates, le sulfate de chondroïtine, le dermatane sulfate, le kératane sulfate, le raffinose, le rhamminose, le mannose, le glucose, le rhamnose, le saccharose, le stachyose, le verbascose, le tréhalose, le lactose, le lactulose, le maltose, l'érythritol, le mannitol, le sorbitol, le xylitol, leurs dérivés et leurs mélanges, des lipides naturels ou synthétiques sélectionnés parmi les cires d'abeille, la cire de candelilla, la cire de son de riz, la cire de carnauba, les cires microcristallines, les huiles animales ou végétales, les huiles animales ou végétales hydrogénées, les phospholipides, les céramides, les esters d'acide gras, leurs dérivés et leurs mélanges, de l'urée, de ses dérivés et leurs mélanges.

Par le terme « support », il est entendu, au sens de la présente invention, un composé ou une molécule, en particulier un composé ou une molécule qui permet la dispersion/la solubilisation /l'intégration en son sein de l'actif (le cannabinoïde non-euphorisant) de telle sorte à former un mélange/une dispersion homogène.

En particulier, selon l'invention, ledit au moins un support est un support thermoplastique, c'est-à-dire un support ayant la propriété de se ramollir lorsqu'il est chauffé suffisamment, mais qui, se refroidissant, redevient dur, friable / cassant.

Selon l'invention, ledit support peut être un polymère, en particulier un polymère thermoplastique.

Au sens de la présente invention, le terme « support » désigne les molécules/composés de type polymère (par exemple les polysaccharides) ou non (par exemples les acides aminés).

Selon un mode de réalisation, il est donc prévu suivant l'invention, une composition sous forme d'un extrudat thermoformé friable / cassant, éventuellement conditionné en pellet, en flake, en granulé, en poudre, en comprimé effervescent ou non, en solution injectable ou non, en suspension, en gel, en pommade ou encore sous toute autre forme adéquate permettant une administration à un animal ou à un être humain, comprenant au moins un cannabinoïde non-euphorisant naturel ou synthétique et au moins un acide aminé, un peptide et/ou un polypeptide naturel ou synthétique en tant que support.

A titre d'exemple, parmi les acides aminés, peuvent être mentionnés les acides aminés suivants : acide aspartique, acide cystéique, acide muramique, acide pyroglutamique, alanine, arginine, asparagine, aspartate, citrulline, cystéine, glutamate, glutamine, glycine, histidine, homocystéine, homosérine, hydroxyproline, isovaline, isoleucine, leucine, lysine, méthionine, methylalanine, norleucine, norvaline, ornithine, phénylalanine, proline, sarcosine, sérine, thréonine, tryptophane, tyrosine, valine, leurs dérivés et leurs mélanges.

A titre d'exemple, parmi les peptides et les polypeptides, peuvent être mentionnés l'aspartame, la carnosine, leurs dérivés et leurs mélanges.

Selon un mode de réalisation, il est donc prévu suivant l'invention, une composition sous forme d'un extrudat thermoformé friable / cassant, éventuellement conditionné en pellet, en flake, en granulé, en poudre, en comprimé effervescent ou non, en solution injectable ou non, en suspension, en gel, en pommade ou encore sous toute autre forme adéquate permettant une administration à un animal ou à un être humain, comprenant au moins un cannabinoïde non-euphorisant naturel ou synthétique et au moins une protéine naturelle ou synthétique en tant que support sélectionnée parmi les glycoprotéines, les collagènes, les hydrolysats de collagènes, les gélatines, les protéines de pois, les protéines de soja, les protéines de blé, les protéines de citrouille, les protéines de noix, les protéines de riz, les protéines de noisette, les protéines de lait, les protéines de petit pois, les protéines de lentilles, les protéines d'amandes, les protéines de pistaches, les protéines de chia, les protéines de chanvre, les protéines de lin, les protéines de courge, les protéines de sésame, les protéines de pavot, les protéines de lupin, les protéines de tournesol, les protéines d'avoine, les protéines de millet, les protéines d'orge, les protéines de seigle, leurs dérivés et leurs mélanges.

Selon un mode de réalisation, il est donc prévu suivant l'invention, une composition sous forme d'un extrudat thermoformé friable / cassant, éventuellement conditionné en pellet, en flake, en granulé, en poudre, en comprimé effervescent ou non, en solution injectable ou non, en suspension, en gel, en pommade ou encore sous toute autre forme adéquate permettant une administration à un animal ou à un être humain, comprenant au moins un cannabinoïde non-euphorisant naturel ou synthétique et au moins un saccharide naturel ou synthétique en tant que support sélectionné parmi l'alginate de calcium, l'alginate de sodium, l'alginate d'ammonium, l'alginate de propylène glycol, les amidons, les amidons prégélatinisés, les amidons modifiés, les fibres solubles ou insolubles, les carraghénanes, la pectine, l'hydroxypropylcellulose (HPC), le glycogène, le β-glucane, l'amylopectine, l'amylose, les dextrines, les cyclodextrines, la maltodextrine, l'isomaltose, l'isomalt, le xylane, le pullulan, l'agar-agar, les mannanes, le fucoïdane, la gomme de xanthane, la gomme de guar, la gomme mastic, la gomme arabique, la gomme d'acacia, la gomme adragante, la gomme ghatti, la gomme de karaya, la gomme de Konjac, la gomme de fenugrec, la gomme d'acajou ou anacarde, la gomme tara, la gomme de caroube, la gomme de cassia, la gomme de benjoin, la gomme cassia, la gomme gaïac, le chitosan, la chitine, la lévane, la néoserine, l'acide hyaluronique, les hyaluronates, le sulfate de chondroïtine, le dermatane sulfate, le kératane sulfate, le raffinose, le rhamminose, le mannose, le glucose, le rhamnose, le saccharose, le stachyose, le verbascose, le tréhalose, le lactose, le lactulose, le maltose, l'érythritol, le mannitol, le sorbitol, le xylitol, leurs dérivés et leurs mélanges.

Le terme « saccharide » regroupe les monosaccharides, les oligosaccharides et les polysaccharides.

A titre d'exemple, parmi les fibres solubles ou insolubles peuvent être mentionnées les fibres de Konjac, les fibres d'acacia, les fibres d'inuline ou encore les fibres d'arabinoxylane. Cette liste est non exhaustive.

Selon un mode de réalisation, il est également prévu suivant l'invention, une composition sous forme d'un extrudat thermoformé friable / cassant, éventuellement conditionné en pellet, en flake, en granulé, en poudre, en comprimé effervescent ou non, en solution injectable ou non, en suspension, en gel, en pommade ou encore sous toute autre forme adéquate permettant une administration à un animal ou à un être humain, comprenant au moins un cannabinoïde non-euphorisant naturel ou synthétique et au moins une dextrine naturelle ou synthétique en tant que support.

A titre d'exemple, parmi les dextrines, peuvent être mentionnés les dextrines de pois, les dextrines de pomme de terre, les dextrines de maïs, leurs dérivés et leurs mélanges. Cette liste est non exhaustive.

Selon un mode de réalisation, il est également prévu suivant l'invention, une composition sous forme d'un extrudat thermoformé friable / cassant, éventuellement conditionné en pellet, en flake, en granulé, en poudre, en comprimé effervescent ou non, en solution injectable ou non, en suspension, en gel, en pommade ou encore sous toute autre forme adéquate permettant une administration à un animal ou à un être humain, comprenant au moins un cannabinoïde non-euphorisant naturel ou synthétique et au moins une cyclodextrine naturelle ou synthétique en tant que support.

A titre d'exemple, parmi les cyclodextrines, peuvent être mentionnés l'a-cyclodextrine, la β-cyclodextrine, la γ-cyclodextrine, leurs dérivés et leurs mélanges. Cette liste est non exhaustive.

Selon un mode de réalisation, il est également prévu suivant l'invention, une composition sous forme d'un extrudat thermoformé friable / cassant, éventuellement conditionné en pellet, en flake, en granulé, en poudre, en comprimé effervescent ou non, en solution injectable ou non, en suspension, en gel, en pommade ou encore sous toute autre forme adéquate permettant une administration à un animal ou à un être humain, comprenant au moins un cannabinoïde non-euphorisant naturel ou synthétique et au moins un hyaluronate naturel ou synthétique en tant que support.

A titre d'exemple, parmi les hyaluronates, peuvent être mentionnés l'acide hyaluronique, l'hyaluronate de sodium, leurs dérivés et leurs mélanges. Cette liste est non exhaustive.

Selon un mode de réalisation, il est également prévu suivant l'invention, une composition sous forme d'un extrudat thermoformé friable / cassant, éventuellement conditionné en pellet, en flake, en granulé, en poudre, en comprimé effervescent ou non, en solution injectable ou non, en suspension, en gel, en pommade ou encore sous toute autre forme adéquate permettant une administration à un animal ou à un être humain, comprenant au moins un cannabinoïde non-euphorisant naturel ou synthétique et au moins une carraghénane naturelle ou synthétique en tant que support.

A titre d'exemple, parmi les carraghénanes, peuvent être mentionnés la κ-carraghénane, la i-carraghénane, la λ-carraghénane, leurs dérivés et leurs mélanges. Cette liste est non exhaustive.

Selon un mode de réalisation, il est également prévu suivant l'invention, une composition sous forme d'un extrudat thermoformé friable / cassant, éventuellement conditionné en pellet, en flake, en granulé, en poudre, en comprimé effervescent ou non, en solution injectable ou non, en suspension, en gel, en pommade ou encore sous toute autre forme adéquate permettant une administration à un animal ou à un être humain, comprenant au moins un cannabinoïde non-euphorisant naturel ou synthétique et au moins un amidon naturel ou synthétique en tant que support.

A titre d'exemple, parmi les amidons peuvent être mentionnés l'amidon de maïs, l'amidon de pomme de terre, l'amidon de riz, l'amidon de blé, l'amidon de sarrasin, l'amidon de châtaigne, l'amidon de patate douce ou encore l'amidon de manioc. Cette liste est non exhaustive.

Selon un mode de réalisation, il est donc prévu suivant l'invention, une composition sous forme d'un extrudat thermoformé friable / cassant, éventuellement conditionné en pellet, en flake, en granulé, en poudre, en comprimé effervescent ou non, en solution injectable ou non, en suspension, en gel, en pommade ou encore sous toute autre forme adéquate permettant une administration à un animal ou à un être humain, comprenant au moins un cannabinoïde non-euphorisant naturel ou synthétique et au moins un lipide naturel ou synthétique en tant que support sélectionné parmi les cires d'abeille, la cire de candelilla, la cire de son de riz, la cire de carnauba, les cires microcristallines, les huiles animales ou végétales, les huiles animales ou végétales hydrogénées, les phospholipides, les céramides, les esters d'acide gras, leurs dérivés et leurs mélanges.

Par le terme « extrudat », il est entendu, au sens de la présente invention, une matière qui sort d'une extrudeuse, en particulier de la filière d'une extrudeuse.

Par les termes « extrudat thermoformé », il est entendu, au sens de la présente invention, une matière qui sort d'un appareillage, en particulier qui sort d'une extrudeuse, dans lequel elle a subi une transformation par l'effet de la chaleur, éventuellement par l'effet combiné de la chaleur et de forces de cisaillement d'une vis sans fin. Une telle transformation par l'effet de la chaleur, éventuellement par l'effet combiné de la chaleur et de forces de cisaillement d'une vis sans fin, peut être obtenue avec la technique de l'extrusion à chaud (HME ou Hot Melt Extrusion).

Un extrudat thermoformé selon l'invention peut donc être obtenu selon la technique d'extrusion à chaud qui permet de réaliser la dispersion moléculaire d'un actif (substance active) au sein d'un support (par exemple au sein d'un polymère / d'une matrice polymérique) pour former des dispersions solides, l'actif et/ou le support mis en œuvre subissant un changement d'état de la matière. L'actif et/ou le support passe donc d'un premier état de la matière à un second état de la matière, lequel second état de la matière impliquant que ledit actif et/ou ledit support se présente au moins partiellement sous forme amorphe. Cette dispersion solide et ce changement d'état de la matière sont possibles grâce à un apport de chaleur et éventuellement grâce à la contrainte appliquée par le mouvement des vis sans fin sur la matière dans un extrudeur. En final, l'extrusion à chaud donne lieu, en sortie, à la formation d'un extrudat thermoformé friable / cassant sous forme d'un jonc qui peut alors notamment être pelletisé ou broyé.

En particulier, un extrudat thermoformé selon l'invention est un extrudat dans lequel un cannabinoïde non-euphorisant et/ou au moins un support naturel ou synthétique choisi dans le groupe constitué des acides aminés, des peptides et des polypeptides naturels ou synthétiques, des protéines naturelles ou synthétiques sélectionnées parmi les glycoprotéines, les collagènes, les hydrolysats de collagènes, les gélatines, les protéines de pois, les protéines de soja, les protéines de blé, les protéines de citrouille, les protéines de noix, les protéines de riz, les protéines de noisette, les protéines de lait, les protéines de petit pois, les protéines de lentilles, les protéines d'amandes, les protéines de pistaches, les protéines de chia, les protéines de chanvre, les protéines de lin, les protéines de courge, les protéines de sésame, les protéines de pavot, les protéines de lupin, les protéines de tournesol, les protéines d'avoine, les protéines de millet, les protéines d'orge, les protéines de seigle, leurs dérivés et leurs mélanges, des saccharides naturels ou synthétiques sélectionnés parmi l'alginate de calcium, l'alginate de sodium, l'alginate d'ammonium, l'alginate de propylène glycol, les amidons, les amidons prégélatinisés, les amidons modifiés, les fibres solubles ou insolubles, les carraghénanes, la pectine, l'hydroxypropylcellulose (HPC), le glycogène, le β-glucane, l'amylopectine, l'amylose, les dextrines, les cyclodextrines, la maltodextrine, l'isomaltose, l'isomalt, le xylane, le pullulan, l'agar-agar, les mannanes, le fucoïdane, la gomme de xanthane, la gomme de guar, la gomme mastic, la gomme arabique, la gomme d'acacia, la gomme adragante, la gomme ghatti, la gomme de karaya, la gomme de Konjac, la gomme de fenugrec, la gomme d'acajou ou anacarde, la gomme tara, la gomme de caroube, la gomme de cassia, la gomme de benjoin, la gomme cassia, la gomme gaïac, le chitosan, la chitine, la lévane, la néoserine, l'acide hyaluronique, les hyaluronates, le sulfate de chondroïtine, le dermatane sulfate, le kératane sulfate, le raffinose, le rhamminose, le mannose, le glucose, le rhamnose, le saccharose, le stachyose, le verbascose, le tréhalose, le lactose, le lactulose, le maltose, l'érythritol, le mannitol, le sorbitol, le xylitol, leurs dérivés et leurs mélanges, des lipides naturels ou synthétiques sélectionnés parmi les cires d'abeille, la cire de candelilla, la cire de son de riz, la cire de carnauba, les cires microcristallines, les huiles animales ou végétales, les huiles animales ou végétales hydrogénées, les phospholipides, les céramides, les esters d'acide gras, leurs dérivés et leurs mélanges, de l'urée, de ses dérivés et leurs mélanges, a/ont été fondu(s) pour donner lieu à une dispersion solide consistant en une structure vitreuse comprenant un mélange moléculaire dudit cannabinoïde non-euphorisant et dudit au moins un support de telle sorte que ledit cannabinoïde non-euphorisant est dispersé au sein dudit au moins un support.

Par les termes « structure vitreuse », il est entendu, au sens de la présente invention, une structure comprenant/ étant formée par un mélange moléculaire d'au moins deux composés/molécules, au moins l'un de ces deux composés/molécules se présentant au moins partiellement sous une forme non cristalline, en particulier se présentant au moins partiellement sous forme amorphe. En particulier, dans le cadre de la présente invention, une telle structure vitreuse est obtenue par un changement d'état de la matière comprenant le principe actif (le cannabinoïde non-euphorisant) et/ou le support mis en oeuvre, lequel changement d'état de la matière est obtenu par extrusion à chaud, la matière étant soumise à un chauffage et subissant une fonte.

Par les termes « dispersion solide », il est entendu, au sens de la présente invention, un mélange/un système d'au moins deux composés/molécules, au moins l'un de ces deux composés/molécules se présentant au moins partiellement sous une forme non cristalline, en particulier se présentant au moins partiellement sous forme amorphe.

En particulier, une « dispersion solide » consiste en une dispersion moléculaire d'un principe actif/d'une substance active au moins partiellement sous forme amorphe au sein d'un support, par exemple au sein d'une matrice polymérique.

Par les termes « extrudat thermoformé friable / cassant », il est entendu, au sens de la présente invention, un extrudat qui se désagrège facilement, qui peut être réduit facilement en poudre et/ou en de petits fragments/morceaux. En particulier, il est entendu, au sens de la présente invention, un extrudat qui ne présente pas de propriétés élastique et collante et qui ne présente pas une texture gommeuse. Un extrudat thermoformé friable / cassant au sens de la présente invention n'est donc pas un extrudat de type chewing-gum.

Selon l'invention, l'extrudat thermoformé ne se présente pas sous forme d'un chewing-gum.

En particulier, un extrudat thermoformé friable / cassant selon l'invention, éventuellement conditionné en pellet, en flake, en granulé, en poudre, en comprimé effervescent ou non, en solution injectable ou non, en suspension, en gel, en pommade ou encore sous toute autre forme adéquate permettant une administration à un animal ou à un être humain, est un extrudat dans lequel le principe actif (ledit au moins un cannabinoïde non-euphorisant naturel ou synthétique) et/ou ledit au moins un support est/sont fondu(s).

En particulier, la composition selon l'invention, plus particulièrement la composition sous forme d'un extrudat thermoformé friable / cassant selon l'invention, éventuellement conditionné en pellet, en flake, en granulé, en poudre, en comprimé effervescent ou non, en solution injectable ou non, en suspension, en gel, en pommade ou encore sous toute autre forme adéquate permettant une administration à un animal ou à un être humain, est une dispersion solide dans laquelle ledit au moins un cannabinoïde non-euphorisant naturel ou synthétique est dispersé dans ledit au moins un support choisi dans le groupe constitué des acides aminés, des peptides et des polypeptides naturels ou synthétiques, des protéines naturelles ou synthétiques sélectionnées parmi les glycoprotéines, les collagènes, les hydrolysats de collagènes, les gélatines, les protéines de pois, les protéines de soja, les protéines de blé, les protéines de citrouille, les protéines de noix, les protéines de riz, les protéines de noisette, les protéines de lait, les protéines de petit pois, les protéines de lentilles, les protéines d'amandes, les protéines de pistaches, les protéines de chia, les protéines de chanvre, les protéines de lin, les protéines de courge, les protéines de sésame, les protéines de pavot, les protéines de lupin, les protéines de tournesol, les protéines d'avoine, les protéines de millet, les protéines d'orge, les protéines de seigle, leurs dérivés et leurs mélanges, des saccharides naturels ou synthétiques sélectionnés parmi l'alginate de calcium, l'alginate de sodium, l'alginate d'ammonium, l'alginate de propylène glycol, les amidons, les amidons prégélatinisés, les amidons modifiés, les fibres solubles ou insolubles, les carraghénanes, la pectine, l'hydroxypropylcellulose (HPC), le glycogène, le β-glucane, l'amylopectine, l'amylose, les dextrines, les cyclodextrines, la maltodextrine, l'isomaltose, l'isomalt, le xylane, le pullulan, l'agar-agar, les mannanes, le fucoïdane, la gomme de xanthane, la gomme de guar, la gomme mastic, la gomme arabique, la gomme d'acacia, la gomme adragante, la gomme ghatti, la gomme de karaya, la gomme de Konjac, la gomme de fenugrec, la gomme d'acajou ou anacarde, la gomme tara, la gomme de caroube, la gomme de cassia, la gomme de benjoin, la gomme cassia, la gomme gaïac, le chitosan, la chitine, la lévane, la néoserine, l'acide hyaluronique, les hyaluronates, le sulfate de chondroïtine, le dermatane sulfate, le kératane sulfate, le raffinose, le rhamminose, le mannose, le glucose, le rhamnose, le saccharose, le stachyose, le verbascose, le tréhalose, le lactose, le lactulose, le maltose, l'érythritol, le mannitol, le sorbitol, le xylitol, leurs dérivés et leurs mélanges, des lipides naturels ou synthétiques sélectionnés parmi les cires d'abeille, la cire de candelilla, la cire de son de riz, la cire de carnauba, les cires microcristallines, les huiles animales ou végétales, les huiles animales ou végétales hydrogénées, les phospholipides, les céramides, les esters d'acide gras, leurs dérivés et leurs mélanges, de l'urée, de ses dérivés et leurs mélanges.

Selon un mode de réalisation, il est donc prévu suivant l'invention, une composition sous forme d'un extrudat thermoformé friable / cassant, éventuellement conditionné en pellet, en flake, en granulé, en poudre, en comprimé effervescent ou non, en solution injectable ou non, en suspension, en gel, en pommade ou encore sous toute autre forme adéquate permettant une administration à un animal ou à un être humain, comprenant au moins un cannabinoïde non-euphorisant naturel ou synthétique et au moins un acide aminé, un peptide et/ou au moins un polypeptide naturel ou synthétique en tant que support, ladite composition étant une dispersion solide dans laquelle ledit au moins un cannabinoïde non-euphorisant naturel ou synthétique est dispersé dans ledit au moins un acide aminé, au moins un peptide et/ou au moins un polypeptide naturel ou synthétique en tant que support, lequel support est/a été fondu.

Selon un mode de réalisation, il est donc prévu suivant l'invention, une composition sous forme d'un extrudat thermoformé friable / cassant, éventuellement conditionné en pellet, en flake, en granulé, en poudre, en comprimé effervescent ou non, en solution injectable ou non, en suspension, en gel, en pommade ou encore sous toute autre forme adéquate permettant une administration à un animal ou à un être humain, comprenant au moins un cannabinoïde non-euphorisant naturel ou synthétique et au moins une protéine naturelle ou synthétique en tant que support sélectionnée parmi les glycoprotéines, les collagènes, les hydrolysats de collagènes, les gélatines, les protéines de pois, les protéines de soja, les protéines de blé, les protéines de citrouille, les protéines de noix, les protéines de riz, les protéines de noisette, les protéines de lait, les protéines de petit pois, les protéines de lentilles, les protéines d'amandes, les protéines de pistaches, les protéines de chia, les protéines de chanvre, les protéines de lin, les protéines de courge, les protéines de sésame, les protéines de pavot, les protéines de lupin, les protéines de tournesol, les protéines d'avoine, les protéines de millet, les protéines d'orge, les protéines de seigle, leurs dérivés et leurs mélanges, ladite composition étant une dispersion solide dans laquelle ledit au moins un cannabinoïde non-euphorisant naturel ou synthétique est dispersé dans ladite au moins une protéine naturelle ou synthétique en tant que support sélectionnée parmi les glycoprotéines, les collagènes, les hydrolysats de collagènes, les gélatines, les protéines de pois, les protéines de soja, les protéines de blé, les protéines de citrouille, les protéines de noix, les protéines de riz, les protéines de noisette, les protéines de lait, les protéines de petit pois, les protéines de lentilles, les protéines d'amandes, les protéines de pistaches, les protéines de chia, les protéines de chanvre, les protéines de lin, les protéines de courge, les protéines de sésame, les protéines de pavot, les protéines de lupin, les protéines de tournesol, les protéines d'avoine, les protéines de millet, les protéines d'orge, les protéines de seigle, leurs dérivés et leurs mélanges, lequel support est/a été fondu.

Selon un mode de réalisation, il est donc prévu suivant l'invention, une composition sous forme d'un extrudat thermoformé friable / cassant, éventuellement conditionné en pellet, en flake, en granulé, en poudre, en comprimé effervescent ou non, en solution injectable ou non, en suspension, en gel, en pommade ou encore sous toute autre forme adéquate permettant une administration à un animal ou à un être humain, comprenant au moins un cannabinoïde non-euphorisant naturel ou synthétique et au moins un saccharide naturel ou synthétique en tant que support sélectionné parmi l'alginate de calcium, l'alginate de sodium, l'alginate d'ammonium, l'alginate de propylène glycol, les amidons, les amidons prégélatinisés, les amidons modifiés, les fibres solubles ou insolubles, les carraghénanes, la pectine, l'hydroxypropylcellulose (HPC), le glycogène, le β-glucane, l'amylopectine, l'amylose, les dextrines, les cyclodextrines, la maltodextrine, l'isomaltose, l'isomalt, le xylane, le pullulan, l'agar-agar, les mannanes, le fucoïdane, la gomme de xanthane, la gomme de guar, la gomme mastic, la gomme arabique, la gomme d'acacia, la gomme adragante, la gomme ghatti, la gomme de karaya, la gomme de Konjac, la gomme de fenugrec, la gomme d'acajou ou anacarde, la gomme tara, la gomme de caroube, la gomme de cassia, la gomme de benjoin, la gomme cassia, la gomme gaïac, le chitosan, la chitine, la lévane, la néoserine, l'acide hyaluronique, les hyaluronates, le sulfate de chondroïtine, le dermatane sulfate, le kératane sulfate, le raffinose, le rhamminose, le mannose, le glucose, le rhamnose, le saccharose, le stachyose, le verbascose, le tréhalose, le lactose, le lactulose, le maltose, l'érythritol, le mannitol, le sorbitol, le xylitol, leurs dérivés et leurs mélanges , ladite composition étant une dispersion solide dans laquelle ledit au moins un cannabinoïde non-euphorisant naturel ou synthétique est dispersé dans ledit au moins un saccharide naturel ou synthétique en tant que support sélectionné parmi l'alginate de calcium, l'alginate de sodium, l'alginate d'ammonium, l'alginate de propylène glycol, les amidons, les amidons prégélatinisés, les amidons modifiés, les fibres solubles ou insolubles, les carraghénanes, la pectine, l'hydroxypropylcellulose (HPC), le glycogène, le β-glucane, l'amylopectine, l'amylose, les dextrines, les cyclodextrines, la maltodextrine, l'isomaltose, l'isomalt, le xylane, le pullulan, l'agar-agar, les mannanes, le fucoïdane, la gomme de xanthane, la gomme de guar, la gomme mastic, la gomme arabique, la gomme d'acacia, la gomme adragante, la gomme ghatti, la gomme de karaya, la gomme de Konjac, la gomme de fenugrec, la gomme d'acajou ou anacarde, la gomme tara, la gomme de caroube, la gomme de cassia, la gomme de benjoin, la gomme cassia, la gomme gaïac, le chitosan, la chitine, la lévane, la néoserine, l'acide hyaluronique, les hyaluronates, le sulfate de chondroïtine, le dermatane sulfate, le kératane sulfate, le raffinose, le rhamminose, le mannose, le glucose, le rhamnose, le saccharose, le stachyose, le verbascose, le tréhalose, le lactose, le lactulose, le maltose, l'érythritol, le mannitol, le sorbitol, le xylitol, leurs dérivés et leurs mélanges, lequel support est/a été fondu.

Selon un mode de réalisation, il est donc prévu suivant l'invention, une composition sous forme d'un extrudat thermoformé friable / cassant, éventuellement conditionné en pellet, en flake, en granulé, en poudre, en comprimé effervescent ou non, en solution injectable ou non, en suspension, en gel, en pommade ou encore sous toute autre forme adéquate permettant une administration à un animal ou à un être humain, comprenant au moins un cannabinoïde non-euphorisant naturel ou synthétique et au moins une dextrine naturelle ou synthétique en tant que support, ladite composition étant une dispersion solide dans laquelle ledit au moins un cannabinoïde non-euphorisant naturel ou synthétique est dispersé dans ladite au moins une dextrine naturelle ou synthétique en tant que support, lequel support est/a été fondu.

Selon un mode de réalisation, il est donc prévu suivant l'invention, une composition sous forme d'un extrudat thermoformé friable / cassant, éventuellement conditionné en pellet, en flake, en granulé, en poudre, en comprimé effervescent ou non, en solution injectable ou non, en suspension, en gel, en pommade ou encore sous toute autre forme adéquate permettant une administration à un animal ou à un être humain, comprenant au moins un cannabinoïde non-euphorisant naturel ou synthétique et au moins une cyclodextrine naturelle ou synthétique en tant que support, ladite composition étant une dispersion solide dans laquelle ledit au moins un cannabinoïde non-euphorisant naturel ou synthétique est dispersé dans ladite au moins une cyclodextrine naturelle ou synthétique en tant que support, lequel support est/a été fondu.

Selon un mode de réalisation, il est donc prévu suivant l'invention, une composition sous forme d'un extrudat thermoformé friable / cassant, éventuellement conditionné en pellet, en flake, en granulé, en poudre, en comprimé effervescent ou non, en solution injectable ou non, en suspension, en gel, en pommade ou encore sous toute autre forme adéquate permettant une administration à un animal ou à un être humain, comprenant au moins un cannabinoïde non-euphorisant naturel ou synthétique et au moins un hyaluronate naturel ou synthétique en tant que support, ladite composition étant une dispersion solide dans laquelle ledit au moins un cannabinoïde non-euphorisant naturel ou synthétique est dispersé dans ledit au moins un hyaluronate naturel ou synthétique en tant que support, lequel support est/a été fondu.

Selon un mode de réalisation, il est donc prévu suivant l'invention, une composition sous forme d'un extrudat thermoformé friable / cassant, éventuellement conditionné en pellet, en flake, en granulé, en poudre, en comprimé effervescent ou non, en solution injectable ou non, en suspension, en gel, en pommade ou encore sous toute autre forme adéquate permettant une administration à un animal ou à un être humain, comprenant au moins un cannabinoïde non-euphorisant naturel ou synthétique et au moins une carraghénane naturelle ou synthétique en tant que support, ladite composition étant une dispersion solide dans laquelle ledit au moins un cannabinoïde non-euphorisant naturel ou synthétique est dispersé dans ladite au moins une carraghénane naturelle ou synthétique en tant que support, lequel support est/a été fondu.

Selon un mode de réalisation, il est donc prévu suivant l'invention, une composition sous forme d'un extrudat thermoformé friable / cassant, éventuellement conditionné en pellet, en flake, en granulé, en poudre, en comprimé effervescent ou non, en solution injectable ou non, en suspension, en gel, en pommade ou encore sous toute autre forme adéquate permettant une administration à un animal ou à un être humain, comprenant au moins un cannabinoïde non-euphorisant naturel ou synthétique et au moins un amidon naturel ou synthétique et/ou au moins un amidon naturel ou synthétique modifié et/ou au moins un amidon naturel ou synthétique prégélatinisé en tant que support, ladite composition étant une dispersion solide dans laquelle ledit au moins un cannabinoïde non-euphorisant naturel ou synthétique est dispersé dans ledit au moins un amidon naturel ou synthétique et/ou dans ledit au moins un amidon naturel ou dans ledit synthétique modifié et/ou dans ledit au moins un amidon naturel ou synthétique prégélatinisé en tant que support, lequel support est/a été fondu.

Selon un mode de réalisation, il est donc prévu suivant l'invention, une composition sous forme d'un extrudat thermoformé friable / cassant, éventuellement conditionné en pellet, en flake, en granulé, en poudre, en comprimé effervescent ou non, en solution injectable ou non, en suspension, en gel, en pommade ou encore sous toute autre forme adéquate permettant une administration à un animal ou à un être humain, comprenant au moins un cannabinoïde non-euphorisant naturel ou synthétique et au moins un lipide naturel ou synthétique en tant que support, ladite composition étant une dispersion solide dans laquelle ledit au moins un cannabinoïde non-euphorisant naturel ou synthétique est dispersé dans ledit au moins un lipide naturel ou synthétique en tant que support sélectionné parmi les cires d'abeille, la cire de candelilla, la cire de son de riz, la cire de carnauba, les cires microcristallines, les huiles animales ou végétales, les huiles animales ou végétales hydrogénées, les phospholipides, les céramides, les esters d'acide gras, leurs dérivés et leurs mélanges, lequel support est/a été fondu.

Un extrudat thermoformé friable / cassant, éventuellement conditionné en pellet, en flake, en granulé, en poudre, en comprimé effervescent ou non, en solution injectable ou non, en suspension, en gel, en pommade ou encore sous toute autre forme adéquate permettant une administration à un animal ou à un être humain, selon l'invention est obtenu par thermoformage à chaud, en particulier par thermoformage à chaud par la technique d'extrusion à chaud. Selon l'invention, le thermoformage à chaud concerne donc plus particulièrement la technique de l'extrusion à chaud.

Avantageusement, selon l'invention, l'extrusion à chaud fait que l'actif (le cannabinoïde non-euphorisant) et le support sont (de préférence simultanément) mélangés, chauffés, fondus, homogénéisés et extrudés. Un mélange intense et une agitation forcée notamment par les vis sans fin pendant le procédé d'extrusion à chaud cause la désagrégation des particules d'actif dans le support fondu donnant lieu à une dispersion solide. La matière (actif + support) se trouve finalement dans un « glassy state » ou « glassy structure » ou état vitreux et se comporte comme un solide friable / cassant, mais sans une structure totalement cristalline et ayant un faible arrangement.

Il est donc prévu selon l'invention une composition obtenue par thermoformage à chaud sous forme d'un extrudat thermoformé friable / cassant, éventuellement conditionné en pellet, en flake, en granulé, en poudre, en comprimé effervescent ou non, en solution injectable ou non, en suspension, en gel, en pommade ou encore sous toute autre forme adéquate permettant une administration à un animal ou à un être humain, obtenu par thermoformage à chaud, en particulier obtenu par extrusion à chaud, ladite composition comprenant au moins un cannabinoïde non-euphorisant naturel ou synthétique et au moins un support naturel ou synthétique choisi dans le groupe constitué des acides aminés, des peptides et des polypeptides naturels ou synthétiques, des protéines naturelles ou synthétiques sélectionnées parmi les glycoprotéines, les collagènes, les hydrolysats de collagènes, les gélatines, les protéines de pois, les protéines de soja, les protéines de blé, les protéines de citrouille, les protéines de noix, les protéines de riz, les protéines de noisette, les protéines de lait, les protéines de petit pois, les protéines de lentilles, les protéines d'amandes, les protéines de pistaches, les protéines de chia, les protéines de chanvre, les protéines de lin, les protéines de courge, les protéines de sésame, les protéines de pavot, les protéines de lupin, les protéines de tournesol, les protéines d'avoine, les protéines de millet, les protéines d'orge, les protéines de seigle, leurs dérivés et leurs mélanges, des saccharides naturels ou synthétiques sélectionnés parmi l'alginate de calcium, l'alginate de sodium, l'alginate d'ammonium, l'alginate de propylène glycol, les amidons, les amidons prégélatinisés, les amidons modifiés, les fibres solubles ou insolubles, les carraghénanes, la pectine, l'hydroxypropylcellulose (HPC), le glycogène, le β-glucane, l'amylopectine, l'amylose, les dextrines, les cyclodextrines, la maltodextrine, l'isomaltose, l'isomalt, le xylane, le pullulan, l'agar-agar, les mannanes, le fucoïdane, la gomme de xanthane, la gomme de guar, la gomme mastic, la gomme arabique, la gomme d'acacia, la gomme adragante, la gomme ghatti, la gomme de karaya, la gomme de Konjac, la gomme de fenugrec, la gomme d'acajou ou anacarde, la gomme tara, la gomme de caroube, la gomme de cassia, la gomme de benjoin, la gomme cassia, la gomme gaïac, le chitosan, la chitine, la lévane, la néoserine, l'acide hyaluronique, les hyaluronates, le sulfate de chondroïtine, le dermatane sulfate, le kératane sulfate, le raffinose, le rhamminose, le mannose, le glucose, le rhamnose, le saccharose, le stachyose, le verbascose, le tréhalose, le lactose, le lactulose, le maltose, l'érythritol, le mannitol, le sorbitol, le xylitol, leurs dérivés et leurs mélanges, des lipides naturels ou synthétiques sélectionnés parmi les cires d'abeille, la cire de candelilla, la cire de son de riz, la cire de carnauba, les cires microcristallines, les huiles animales ou végétales, les huiles animales ou végétales hydrogénées, les phospholipides, les céramides, les esters d'acide gras, leurs dérivés et leurs mélanges, de l'urée, de ses dérivés et leurs mélanges.

Un extrudat thermoformé friable / cassant selon l'invention, éventuellement conditionné en pellet, en flake, en granulé, en poudre, en comprimé effervescent ou non, en solution injectable ou non, en suspension, en gel, en pommade ou encore sous toute autre forme adéquate permettant une administration à un animal ou à un être humain, peut donc être obtenu selon la technique d'extrusion à chaud qui permet de réaliser la dispersion moléculaire d'un actif (substance active) au sein d'un support, en particulier au sein d'une matrice polymérique (au sein d'un polymère), pour former des dispersions solides. Cette dispersion solide est possible grâce à un apport de chaleur et éventuellement grâce à la contrainte appliquée par le mouvement des vis sans fin sur la matière dans un extrudeur. En final, l'extrusion à chaud donne lieu, en sortie, à la formation d'un extrudat thermoformé friable / cassant sous forme d'un jonc qui peut alors notamment être pelletisé ou broyé.

La technique d'extrusion à chaud peut être réalisée sans apport d'une phase liquide mais repose sur un apport de chaleur (chauffage) pour assurer une transformation de la matière (changement d'état de la matière) par thermoformage. La technique d'extrusion à chaud donne lieu à une transformation de la matière (changement d'état de la matière), en particulier à une structure vitreuse (« glassy structure ») obtenue sous l'action de la chaleur (chauffage), les particules constituant les poudres n'étant plus toutes présentes sous leur forme initiale (native) cristalline en fin de procédé d'extrusion à chaud mais ayant subis une transformation par thermoformage.

Selon l'invention, de préférence, ledit au moins un cannabinoïde non-euphorisant naturel ou synthétique comprend au moins une première phase amorphe et éventuellement une deuxième phase cristalline.

Par les termes « comprend au moins une première phase amorphe et éventuellement une deuxième phase cristalline », il est entendu, au sens de la présente invention, que ledit au moins un cannabinoïde non-euphorisant naturel ou synthétique peut soit comprendre 100% en masse d'une phase amorphe, soit qu'il peut comprendre simultanément une première phase amorphe et une deuxième phase cristalline, la somme des pourcentages en masse des première et deuxième phases étant dans ce cas égale à 100. En d'autres termes, la composition selon l'invention peut comprendre ledit au moins un cannabinoïde non-euphorisant naturel ou synthétique (1) totalement sous forme amorphe ou (2) partiellement sous forme (phase) amorphe et partiellement sous forme (phase) cristalline.

Notons qu'une phase est dite amorphe lorsque les atomes la constituant ne respectent aucun ordre à moyenne et grande distance, ce qui la distingue d'une phase dite cristalline.

Dans le cadre de la présente invention, une analyse par calorimétrie différentielle à balayage (DSC) qui est bien connue de l'homme de métier peut être utilisée afin de mesurer la différence d'échange de chaleur entre une référence et un produit étudié. Cette technique permet de déterminer les températures de fusion, les températures de cristallisation ou les températures de transitions de phase mais aussi les enthalpies de réaction. En particulier, une telle analyse permet d'obtenir des courbes où la présence d'un pic endothermique (absorption de la chaleur) montre la fusion de l'échantillon analysé. Un pic de fusion indique la présence d'une forme cristalline dans l'échantillon. Au contraire, lorsque le pic de fusion n'est pas présent, le matériel est à l'état amorphe.

La composition selon l'invention se présente donc de préférence sous forme d'un extrudat thermoformé friable / cassant, éventuellement conditionné en pellet, en flake, en granulé, en poudre, en comprimé effervescent ou non, en solution injectable ou non, en suspension, en gel, en pommade ou encore sous toute autre forme adéquate permettant une administration à un animal ou à un être humain, dans lequel ledit au moins un cannabinoïde non-euphorisant naturel ou synthétique (principe actif) comprend au moins une première phase amorphe et éventuellement une deuxième phase cristalline, laquelle ou lesquelles phases(s) est/sont dispersée(s) au sein d'au moins un support choisi dans le groupe constitué des acides aminés, des peptides et des polypeptides naturels ou synthétiques, des protéines naturelles ou synthétiques sélectionnées parmi les glycoprotéines, les collagènes, les hydrolysats de collagènes, les gélatines, les protéines de pois, les protéines de soja, les protéines de blé, les protéines de citrouille, les protéines de noix, les protéines de riz, les protéines de noisette, les protéines de lait, les protéines de petit pois, les protéines de lentilles, les protéines d'amandes, les protéines de pistaches, les protéines de chia, les protéines de chanvre, les protéines de lin, les protéines de courge, les protéines de sésame, les protéines de pavot, les protéines de lupin, les protéines de tournesol, les protéines d'avoine, les protéines de millet, les protéines d'orge, les protéines de de seigle, leurs dérivés et leurs mélanges, des saccharides naturels ou synthétiques sélectionnés parmi l'alginate de calcium, l'alginate de sodium, l'alginate d'ammonium, l'alginate de propylène glycol, les amidons, les amidons prégélatinisés, les amidons modifiés, les fibres solubles ou insolubles, les carraghénanes, la pectine, l'hydroxypropylcellulose (HPC), le glycogène, le β-glucane, l'amylopectine, l'amylose, les dextrines, les cyclodextrines, la maltodextrine, l'isomaltose, l'isomalt, le xylane, le pullulan, l'agar-agar, les mannanes, le fucoïdane, la gomme de xanthane, la gomme de guar, la gomme mastic, la gomme arabique, la gomme d'acacia, la gomme adragante, la gomme ghatti, la gomme de karaya, la gomme de Konjac, la gomme de fenugrec, la gomme d'acajou ou anacarde, la gomme tara, la gomme de caroube, la gomme de cassia, la gomme de benjoin, la gomme cassia, la gomme gaïac, le chitosan, la chitine, la lévane, la néoserine, l'acide hyaluronique, les hyaluronates, le sulfate de chondroïtine, le dermatane sulfate, le kératane sulfate, le raffinose, le rhamminose, le mannose, le glucose, le rhamnose, le saccharose, le stachyose, le verbascose, le tréhalose, le lactose, le lactulose, le maltose, l'érythritol, le mannitol, le sorbitol, le xylitol, leurs dérivés et leurs mélanges, des lipides naturels ou synthétiques sélectionnés parmi les cires d'abeille, la cire de candelilla, la cire de son de riz, la cire de carnauba, les cires microcristallines, les huiles animales ou végétales, les huiles animales ou végétales hydrogénées, les phospholipides, les céramides, les esters d'acide gras, leurs dérivés et leurs mélanges, de l'urée, de ses dérivés et leurs mélanges.

Par les termes « acide aminé naturel », il est entendu au sens de la présente invention tout acide aminé naturellement présent dans le monde du vivant, en particulier un acide aminé végétal ou animal.

Par les termes « acide aminé synthétique », il est entendu au sens de la présente invention tout acide aminé faisant l'objet d'une intervention humaine, en particulier un acide aminé obtenu au départ d'un procédé chimique ou biochimique ou biotechnologique.

Par les termes « peptide ou polypeptide naturel », il est entendu au sens de la présente invention tout peptide ou polypeptide naturellement présent dans le monde du vivant, en particulier un peptide ou un polypeptide végétal ou animal.

Par les termes « peptide ou polypeptide synthétique », il est entendu au sens de la présente invention tout peptide ou polypeptide faisant l'objet d'une intervention humaine, en particulier un peptide ou un polypeptide obtenu au départ d'un procédé chimique ou biochimique ou biotechnologique.

Par les termes « protéine naturelle », il est entendu au sens de la présente invention toute protéine naturellement présente dans le monde du vivant, en particulier une protéine végétale ou animale.

Par les termes « protéine synthétique », il est entendu au sens de la présente invention toute protéine faisant l'objet d'une intervention humaine, en particulier une protéine obtenue au départ d'un procédé chimique ou biochimique ou biotechnologique.

Par les termes « saccharide naturel », il est entendu au sens de la présente invention tout saccharide naturellement présent dans le monde du vivant, en particulier un saccharide végétal ou animal.

Par les termes « saccharide synthétique », il est entendu au sens de la présente invention tout saccharide faisant l'objet d'une intervention humaine, en particulier tout saccharide obtenu au départ d'un procédé chimique ou biochimique ou biotechnologique.

Le terme « saccharide » regroupe toute forme de saccharide dont les monosaccharides, les oligosaccharides et les polysaccharides.

Par les termes « lipide naturel », il est entendu au sens de la présente invention tout lipide naturellement présent dans le monde du vivant, en particulier un lipide végétal.

Par les termes « lipide synthétique », il est entendu au sens de la présente invention tout lipide faisant l'objet d'une intervention humaine, en particulier un lipide obtenu au départ d'un procédé chimique ou biochimique ou biotechnologique.

Par les termes « alginate naturel », il est entendu au sens de la présente invention tout alginate naturellement présent dans le monde du vivant, en particulier un alginate végétal ou animal.

Par les termes « alginate synthétique », il est entendu au sens de la présente invention tout alginate faisant l'objet d'une intervention humaine, en particulier tout alginate obtenu au départ d'un procédé chimique ou biochimique ou biotechnologique.

Par les termes « polysaccharide naturel », il est entendu au sens de la présente invention tout polysaccharide naturellement présent dans le monde du vivant, en particulier un polysaccharide végétal ou animal.

Par les termes « polysaccharide synthétique », il est entendu au sens de la présente invention tout polysaccharide faisant l'objet d'une intervention humaine, en particulier tout polysaccharide obtenu au départ d'un procédé chimique ou biochimique ou biotechnologique.

Par les termes « oligosaccharide naturel », il est entendu au sens de la présente invention tout oligosaccharide naturellement présent dans le monde du vivant, en particulier un oligosaccharide végétal ou animal.

Par les termes « oligosaccharide synthétique », il est entendu au sens de la présente invention tout oligosaccharide faisant l'objet d'une intervention humaine, en particulier tout oligosaccharide obtenu au départ d'un procédé chimique ou biochimique ou biotechnologique.

Par les termes « dextrine naturelle », il est entendu au sens de la présente invention toute dextrine naturellement présente dans le monde du vivant, en particulier une dextrine végétale ou animale.

Par les termes « dextrine synthétique », il est entendu au sens de la présente invention toute dextrine faisant l'objet d'une intervention humaine, en particulier une dextrine obtenue au départ d'un procédé chimique ou biochimique ou biotechnologique.

Par les termes « cyclodextrine naturelle », il est entendu au sens de la présente invention toute cyclodextrine naturellement présente dans le monde du vivant, en particulier une cyclodextrine végétale ou animale.

Par les termes « cyclodextrine synthétique », il est entendu au sens de la présente invention toute cyclodextrine faisant l'objet d'une intervention humaine, en particulier une cyclodextrine obtenue au départ d'un procédé chimique ou biochimique ou biotechnologique.

Par les termes « hyaluronate naturel », il est entendu au sens de la présente invention tout hyaluronate naturellement présent dans le monde du vivant, en particulier un hyaluronate végétal ou animal.

Par les termes « hyaluronate synthétique », il est entendu au sens de la présente invention tout hyaluronate faisant l'objet d'une intervention humaine, en particulier tout hyaluronate obtenu au départ d'un procédé chimique ou biochimique ou biotechnologique.

Par les termes « carraghénane naturelle », il est entendu au sens de la présente invention toute carraghénane naturellement présente dans le monde du vivant, en particulier une carraghénane végétale.

Par les termes « carraghénane synthétique », il est entendu au sens de la présente invention toute carraghénane faisant l'objet d'une intervention humaine, en particulier une carraghénane obtenue au départ d'un procédé chimique ou biochimique ou biotechnologique.

Par les termes « amidon naturel », il est entendu au sens de la présente invention tout amidon naturellement présent dans le monde du vivant, en particulier un amidon végétal ou animal.

Par les termes « amidon synthétique », il est entendu au sens de la présente invention tout amidon faisant l'objet d'une intervention humaine, en particulier tout amidon obtenu au départ d'un procédé chimique ou biochimique ou biotechnologique.

Il a été déterminé, dans le cadre de la présente invention, qu'une telle composition sous forme d'un extrudat thermoformé friable / cassant, éventuellement conditionné en pellet, en flake, en granulé, en poudre, en comprimé effervescent ou non, en solution injectable ou non, en suspension, en gel, en pommade ou encore sous toute autre forme adéquate permettant une administration à un animal ou à un être humain, comprenant au moins un cannabinoïde non-euphorisant naturel ou synthétique comme principe actif et au moins support choisi dans le groupe constitué des acides aminés, des peptides et des polypeptides naturels ou synthétiques, des protéines naturelles ou synthétiques sélectionnées parmi les glycoprotéines, les collagènes, les hydrolysats de collagènes, les gélatines, les protéines de pois, les protéines de soja, les protéines de blé, les protéines de citrouille, les protéines de noix, les protéines de riz, les protéines de noisette, les protéines de lait, les protéines de petit pois, les protéines de lentilles, les protéines d'amandes, les protéines de pistaches, les protéines de chia, les protéines de chanvre, les protéines de lin, les protéines de courge, les protéines de sésame, les protéines de pavot, les protéines de lupin, les protéines de tournesol, les protéines d'avoine, les protéines de millet, les protéines d'orge, les protéines de seigle, leurs dérivés et leurs mélanges, des saccharides naturels ou synthétiques sélectionnés parmi l'alginate de calcium, l'alginate de sodium, l'alginate d'ammonium, l'alginate de propylène glycol, les amidons, les amidons prégélatinisés, les amidons modifiés, les fibres solubles ou insolubles, les carraghénanes, la pectine, l'hydroxypropylcellulose (HPC), le glycogène, le β-glucane, l'amylopectine, l'amylose, les dextrines, les cyclodextrines, la maltodextrine, l'isomaltose, l'isomalt, le xylane, le pullulan, l'agar-agar, les mannanes, le fucoïdane, la gomme de xanthane, la gomme de guar, la gomme mastic, la gomme arabique, la gomme d'acacia, la gomme adragante, la gomme ghatti, la gomme de karaya, la gomme de Konjac, la gomme de fenugrec, la gomme d'acajou ou anacarde, la gomme tara, la gomme de caroube, la gomme de cassia, la gomme de benjoin, la gomme cassia, la gomme gaïac, le chitosan, la chitine, la lévane, la néoserine, l'acide hyaluronique, les hyaluronates, le sulfate de chondroïtine, le dermatane sulfate, le kératane sulfate, le raffinose, le rhamminose, le mannose, le glucose, le rhamnose, le saccharose, le stachyose, le verbascose, le tréhalose, le lactose, le lactulose, le maltose, l'érythritol, le mannitol, le sorbitol, le xylitol, leurs dérivés et leurs mélanges, des lipides naturels ou sélectionnés parmi les cires d'abeille, la cire de candelilla, la cire de son de riz, la cire de carnauba, les cires microcristallines, les huiles animales ou végétales, les huiles animales ou végétales hydrogénées, les phospholipides, les céramides, les esters d'acide gras, leurs dérivés et leurs mélanges, de l'urée, de ses dérivés et leurs mélanges, présente une solubilité et/ou une dispersion en phase aqueuse (milieu aqueux) nettement supérieure dudit au moins un cannabinoïde non-euphorisant naturel ou synthétique. Par ailleurs, il a été montré qu'une telle composition selon l'invention présente une biodisponibilité/bioaccessibilité significativement augmentée dudit au moins un cannabinoïde non-euphorisant naturel ou synthétique par rapport aux biodisponibilités/bioaccessibilités observées pour les compositions actuelles.

Selon l'invention, le principe actif, c'est-à-dire ledit au moins un cannabinoïde non-euphorisant naturel ou synthétique est dispersé/réparti/distribué de manière homogène au sein d'au moins un support choisi dans le groupe constitué des acides aminés, des peptides et des polypeptides naturels ou synthétiques, des protéines naturelles ou synthétiques sélectionnées parmi les glycoprotéines, les collagènes, les hydrolysats de collagènes, les gélatines, les protéines de pois, les protéines de soja, les protéines de blé, les protéines de citrouille, les protéines de noix, les protéines de riz, les protéines de noisette, les protéines de lait, les protéines de petit pois, les protéines de lentilles, les protéines d'amandes, les protéines de pistaches, les protéines de chia, les protéines de chanvre, les protéines de lin, les protéines de courge, les protéines de sésame, les protéines de pavot, les protéines de lupin, les protéines de tournesol, les protéines d'avoine, les protéines de millet, les protéines d'orge, les protéines de seigle, leurs dérivés et leurs mélanges, des saccharides naturels ou synthétiques sélectionnés parmi l'alginate de calcium, l'alginate de sodium, l'alginate d'ammonium, l'alginate de propylène glycol, les amidons, les amidons prégélatinisés, les amidons modifiés, les fibres solubles ou insolubles, les carraghénanes, la pectine, l'hydroxypropylcellulose (HPC), le glycogène, le β-glucane, l'amylopectine, l'amylose, les dextrines, les cyclodextrines, la maltodextrine, l'isomaltose, l'isomalt, le xylane, le pullulan, l'agar-agar, les mannanes, le fucoïdane, la gomme de xanthane, la gomme de guar, la gomme mastic, la gomme arabique, la gomme d'acacia, la gomme adragante, la gomme ghatti, la gomme de karaya, la gomme de Konjac, la gomme de fenugrec, la gomme d'acajou ou anacarde, la gomme tara, la gomme de caroube, la gomme de cassia, la gomme de benjoin, la gomme cassia, la gomme gaïac, le chitosan, la chitine, la lévane, la néoserine, l'acide hyaluronique, les hyaluronates, le sulfate de chondroïtine, le dermatane sulfate, le kératane sulfate, le raffinose, le rhamminose, le mannose, le glucose, le rhamnose, le saccharose, le stachyose, le verbascose, le tréhalose, le lactose, le lactulose, le maltose, l'érythritol, le mannitol, le sorbitol, le xylitol, leurs dérivés et leurs mélanges, des lipides naturels ou synthétiques sélectionnés parmi les cires d'abeille, la cire de candelilla, la cire de son de riz, la cire de carnauba, les cires microcristallines, les huiles animales ou végétales, les huiles animales ou végétales hydrogénées, les phospholipides, les céramides, les esters d'acide gras, leurs dérivés et leurs mélanges, de l'urée, de ses dérivés et leurs mélanges, le principe actif et/ou ledit au moins un support étant fondus lors du procédé de fabrication par thermoformage mis en œuvre selon l'invention et décrit plus loin.

En outre, une composition selon l'invention peut être conservée plusieurs mois sans que ses propriétés ne soient altérées. En particulier, il a été mis en évidence qu'une composition selon l'invention conserve ses propriétés en termes de solubilité et/ou de dispersion en phase aqueuse (milieu aqueux) dudit au moins un cannabinoïde non-euphorisant naturel ou synthétique et en termes de taux de relargage de ce composé au cours du temps au départ d'une composition/formulation selon l'invention, ceci en particulier en phase aqueuse.

Avantageusement, selon l'invention, ledit extrudat thermoformé friable / cassant, éventuellement conditionné en pellet, en flake, en granulé, en poudre, en comprimé effervescent ou non, en solution injectable ou non, en suspension, en gel, en pommade ou encore sous toute autre forme adéquate permettant une administration à un animal ou à un être humain, comprend un mélange thermoformé d'au moins un cannabinoïde non-euphorisant naturel ou synthétique et d'au moins un support naturel ou synthétique choisi dans le groupe constitué des acides aminés, des peptides et des polypeptides naturels ou synthétiques, des protéines naturelles ou synthétiques sélectionnées parmi les glycoprotéines, les collagènes, les hydrolysats de collagènes, les gélatines, les protéines de pois, les protéines de soja, les protéines de blé, les protéines de citrouille, les protéines de noix, les protéines de riz, les protéines de noisette, les protéines de lait, les protéines de petit pois, les protéines de lentilles, les protéines d'amandes, les protéines de pistaches, les protéines de chia, les protéines de chanvre, les protéines de lin, les protéines de courge, les protéines de sésame, les protéines de pavot, les protéines de lupin, les protéines de tournesol, les protéines d'avoine, les protéines de millet, les protéines d'orge, les protéines de seigle, leurs dérivés et leurs mélanges, des saccharides naturels ou synthétiques sélectionnés parmi l'alginate de calcium, l'alginate de sodium, l'alginate d'ammonium, l'alginate de propylène glycol, les amidons, les amidons prégélatinisés, les amidons modifiés, les fibres solubles ou insolubles, les carraghénanes, la pectine, l'hydroxypropylcellulose (HPC), le glycogène, le β-glucane, l'amylopectine, l'amylose, les dextrines, les cyclodextrines, la maltodextrine, l'isomaltose, l'isomalt, le xylane, le pullulan, l'agar-agar, les mannanes, le fucoïdane, la gomme de xanthane, la gomme de guar, la gomme mastic, la gomme arabique, la gomme d'acacia, la gomme adragante, la gomme ghatti, la gomme de karaya, la gomme de Konjac, la gomme de fenugrec, la gomme d'acajou ou anacarde, la gomme tara, la gomme de caroube, la gomme de cassia, la gomme de benjoin, la gomme cassia, la gomme gaïac, le chitosan, la chitine, la lévane, la néoserine, l'acide hyaluronique, les hyaluronates, le sulfate de chondroïtine, le dermatane sulfate, le kératane sulfate, le raffinose, le rhamminose, le mannose, le glucose, le rhamnose, le saccharose, le stachyose, le verbascose, le tréhalose, le lactose, le lactulose, le maltose, l'érythritol, le mannitol, le sorbitol, le xylitol, leurs dérivés et leurs mélanges, des lipides naturels ou synthétiques sélectionnés parmi les cires d'abeille, la cire de candelilla, la cire de son de riz, la cire de carnauba, les cires microcristallines, les huiles animales ou végétales, les huiles animales ou végétales hydrogénées, les phospholipides, les céramides, les esters d'acide gras, leurs dérivés et leurs mélanges, de l'urée, de ses dérivés et leurs mélanges.

Alternativement, selon l'invention, ledit extrudat thermoformé friable / cassant, éventuellement conditionné en pellet, en flake, en granulé, en poudre, en comprimé effervescent ou non, en solution injectable ou non, en suspension, en gel, en pommade ou encore sous toute autre forme adéquate permettant une administration à un animal ou à un être humain, est constitué d'un mélange thermoformé d'au moins un cannabinoïde non-euphorisant naturel ou synthétique et d'au moins un support choisi dans le groupe constitué des acides aminés, des peptides et des polypeptides naturels ou synthétiques, des protéines naturelles ou synthétiques sélectionnées parmi les glycoprotéines, les collagènes, les hydrolysats de collagènes, les gélatines, les protéines de pois, les protéines de soja, les protéines de blé, les protéines de citrouille, les protéines de noix, les protéines de riz, les protéines de noisette, les protéines de lait, les protéines de petit pois, les protéines de lentilles, les protéines d'amandes, les protéines de pistaches, les protéines de chia, les protéines de chanvre, les protéines de lin, les protéines de courge, les protéines de sésame, les protéines de pavot, les protéines de lupin, les protéines de tournesol, les protéines d'avoine, les protéines de millet, les protéines d'orge, les protéines de seigle, leurs dérivés et leurs mélanges, des saccharides naturels ou synthétiques sélectionnés parmi l'alginate de calcium, l'alginate de sodium, l'alginate d'ammonium, l'alginate de propylène glycol, les amidons, les amidons prégélatinisés, les amidons modifiés, les fibres solubles ou insolubles, les carraghénanes, la pectine, l'hydroxypropylcellulose (HPC), le glycogène, le β-glucane, l'amylopectine, l'amylose, les dextrines, les cyclodextrines, la maltodextrine, l'isomaltose, l'isomalt, le xylane, le pullulan, l'agar-agar, les mannanes, le fucoïdane, la gomme de xanthane, la gomme de guar, la gomme mastic, la gomme arabique, la gomme d'acacia, la gomme adragante, la gomme ghatti, la gomme de karaya, la gomme de Konjac, la gomme de fenugrec, la gomme d'acajou ou anacarde, la gomme tara, la gomme de caroube, la gomme de cassia, la gomme de benjoin, la gomme cassia, la gomme gaïac, le chitosan, la chitine, la lévane, la néoserine, l'acide hyaluronique, les hyaluronates, le sulfate de chondroïtine, le dermatane sulfate, le kératane sulfate, le raffinose, le rhamminose, le mannose, le glucose, le rhamnose, le saccharose, le stachyose, le verbascose, le tréhalose, le lactose, le lactulose, le maltose, l'érythritol, le mannitol, le sorbitol, le xylitol, leurs dérivés et leurs mélanges, des lipides naturels ou synthétiques sélectionnés parmi les cires d'abeille, la cire de candelilla, la cire de son de riz, la cire de carnauba, les cires microcristallines, les huiles animales ou végétales, les huiles animales ou végétales hydrogénées, les phospholipides, les céramides, les esters d'acide gras, leurs dérivés et leurs mélanges, de l'urée, de ses dérivés et leurs mélanges.

Il est donc prévu selon l'invention un extrudat thermoformé friable / cassant, éventuellement conditionné en pellet, en flake, en granulé, en poudre, en comprimé effervescent ou non, en solution injectable ou non, en suspension, en gel, en pommade ou encore sous toute autre forme adéquate permettant une administration à un animal ou à un être humain, obtenu par thermoformage à chaud, en particulier obtenu par extrusion à chaud, ledit extrudat thermoformé friable / cassant comprenant un mélange thermoformé d'au moins un cannabinoïde non-euphorisant naturel ou synthétique et d'au moins un support choisi dans le groupe constitué des acides aminés, des peptides et des polypeptides naturels ou synthétiques, des protéines naturelles ou synthétiques sélectionnées parmi les glycoprotéines, les collagènes, les hydrolysats de collagènes, les gélatines, les protéines de pois, les protéines de soja, les protéines de blé, les protéines de citrouille, les protéines de noix, les protéines de riz, les protéines de noisette, les protéines de lait, les protéines de petit pois, les protéines de lentilles, les protéines d'amandes, les protéines de pistaches, les protéines de chia, les protéines de chanvre, les protéines de lin, les protéines de courge, les protéines de sésame, les protéines de pavot, les protéines de lupin, les protéines de tournesol, les protéines d'avoine, les protéines de millet, les protéines d'orge, les protéines de seigle, leurs dérivés et leurs mélanges, des saccharides naturels ou synthétiques sélectionnés parmi l'alginate de calcium, l'alginate de sodium, l'alginate d'ammonium, l'alginate de propylène glycol, les amidons, les amidons prégélatinisés, les amidons modifiés, les fibres solubles ou insolubles, les carraghénanes, la pectine, l'hydroxypropylcellulose (HPC), le glycogène, le β-glucane, l'amylopectine, l'amylose, les dextrines, les cyclodextrines, la maltodextrine, l'isomaltose, l'isomalt, le xylane, le pullulan, l'agar-agar, les mannanes, le fucoïdane, la gomme de xanthane, la gomme de guar, la gomme mastic, la gomme arabique, la gomme d'acacia, la gomme adragante, la gomme ghatti, la gomme de karaya, la gomme de Konjac, la gomme de fenugrec, la gomme d'acajou ou anacarde, la gomme tara, la gomme de caroube, la gomme de cassia, la gomme de benjoin, la gomme cassia, la gomme gaïac, le chitosan, la chitine, la lévane, la néoserine, l'acide hyaluronique, les hyaluronates, le sulfate de chondroïtine, le dermatane sulfate, le kératane sulfate, le raffinose, le rhamminose, le mannose, le glucose, le rhamnose, le saccharose, le stachyose, le verbascose, le tréhalose, le lactose, le lactulose, le maltose, l'érythritol, le mannitol, le sorbitol, le xylitol, leurs dérivés et leurs mélanges, des lipides naturels ou synthétiques sélectionnés parmi les cires d'abeille, la cire de candelilla, la cire de son de riz, la cire de carnauba, les cires microcristallines, les huiles animales ou végétales, les huiles animales ou végétales hydrogénées, les phospholipides, les céramides, les esters d'acide gras, leurs dérivés et leurs mélanges, de l'urée, de ses dérivés et leurs mélanges.

Par les termes « mélange thermoformé », il est entendu, au sens de la présente invention, un mélange qui sort d'un appareillage, en particulier qui sort d'une extrudeuse, dans lequel il a subi une transformation par l'effet de la chaleur, éventuellement par l'effet combiné de la chaleur et de forces de cisaillement d'une vis sans fin. Une telle transformation par l'effet de la chaleur, éventuellement par l'effet combiné de la chaleur et de forces de cisaillement d'une vis sans fin, peut être obtenue avec la technique de l'extrusion à chaud (HME).

En particulier, un mélange thermoformé selon l'invention est un mélange dans lequel le principe actif (ledit au moins un cannabinoïde non-euphorisant naturel ou synthétique) et/ou ledit au moins un support est/sont fondu(s) / a été/ont été fondu(s).

Un mélange thermoformé selon l'invention peut donc être obtenu selon la technique d'extrusion à chaud qui permet de réaliser la dispersion moléculaire d'un actif (substance active) au sein d'un support, par exemple au sein d'une matrice polymérique (au sein d'un polymère) pour former des dispersions solides, l'actif et/ou le support mis en œuvre subissant un changement d'état de la matière. L'actif et/ou le polymère passe donc d'un premier état de la matière à un second état de la matière, lequel second état de la matière impliquant que ledit actif et/ou ledit support se présente au moins partiellement sous forme amorphe.

Selon l'invention, le dit au moins un cannabinoïde non-euphorisant naturel ou synthétique comprend une première phase amorphe.

Avantageusement, selon l'invention, ledit au moins un cannabinoïde non-euphorisant naturel ou synthétique comprend majoritairement au moins une première phase amorphe.

De préférence, selon l'invention, ledit au moins un cannabinoïde non-euphorisant naturel ou synthétique comprend entre 51 et 100% en masse d'une phase amorphe et entre 0 et 49% d'une phase cristalline.

Par les termes « majoritairement au moins une première phase amorphe », il est donc entendu, au sens de la présente invention, que ledit au moins un cannabinoïde non-euphorisant naturel ou synthétique comprend entre 50 et 100% en masse d'une phase amorphe et entre 0 et 50% d'une phase cristalline, plus particulièrement que ledit au moins un cannabinoïde non-euphorisant naturel ou synthétique comprend entre 51 et 100% en masse d'une phase amorphe et entre 0 et 49% d'une phase cristalline.

De préférence, selon l'invention, ledit au moins un cannabinoïde non-euphorisant naturel ou synthétique est choisi dans le groupe constitué du cannabidiol (CBD), du cannabigérol (CBG), du cannabichromène (CBC), de la cannabidivarine (CBDV), de l'acide cannabidiolique (CBDA), de la cannabigérovarine (CBGV), de l'acide cannabichroménique (CBCA), de l'acide cannabiorcichroménique, de l'acide 8-chloro-cannabiorcichroménique, de l'acide cannachromévarinique (CBCVA), de la cannabielsoïne (CBE), du cannabicyclol (CBL), du perrottetinene, de l'acide perrottittenique, de l'acide lunularique, de la vitatine, de l'helichrysum CBG, des amorfrutines (A, B, C et D), des machaeridiols (A, B, C), de l'acide daurichroménique, de l'acide anthopogocyclolique, de l'acide anthopogochroménique, de l'acide geranyl-orsellinique, de la confluentine, de l'acide griffolique, du rubiginosin A, de leurs dérivés et de leurs mélanges.

A titre d'exemple, lorsque ladite au moins une protéine naturelle ou synthétique est du collagène ou de la gélatine, il peut s'agir de collagène ou de gélatine d'origine animale (poisson, porc, bœuf, ...).

En particulier et avantageusement selon l'invention, lesdits hydrolysats de collagène sont des hydrolysats de collagène n'ayant pas / ne présentant pas la capacité de gélifier, c'est-à-dire des hydrolysats de collagène non gélifiants.

Le terme « protéine » désigne une macromolécule constituée de différents acides aminés qui sont liés entre eux. La protéine est l'élément le plus commun et l'unité moléculaire de base de tout être vivant. Les protéines sont caractérisées par la longueur de leur chaîne moléculaire.

Le terme « acide aminé » désigne un acide carboxylique qui possède également un groupe fonctionnel amine et le terme « peptide » désigne un polymère d'acides-aminés.

Le terme « collagène » désigne une protéine composée de trois chaînes alpha polypeptidiques associées. Ces chaînes sont reliées par des liaisons hydrogène entre l'hydroxylysine et l'hydroxyproline et des liaisons covalentes.

Le terme « gélatine » désigne du collagène qui a été modifié, les chaînes moléculaires restant de préférence suffisamment longues pour assurer les propriétés fonctionnelles recherchées avec la gélatine (viscosité, tenue de la gelée).

Les termes « peptides de collagène » désignent de petits groupements ordonnés d'acides aminés issus de la fragmentation de la molécule de collagène. En fonction de la technique d'hydrolyse utilisée, ces groupements sont plus ou moins important et plus ou moins actifs.

Les termes « collagène hydrolysé » indiquent que le collagène a été hydrolysé, notamment pour le rendre plus assimilable.

Préférentiellement, selon l'invention, lesdits collagènes et/ou lesdits hydrolysats de collagène et/ou lesdites gélatines présentent un poids moléculaire compris entre 50 et 300000 Da, de préférence entre 100 et 275000 Da, de préférence entre 150 et 250000 Da, de préférence entre 200 et 225000 Da, de préférence entre 250 et 200000 Da, de préférence entre 300 et 175000 Da, de préférence entre 350 et 150000 Da, de préférence entre 400 et 125000 Da, de préférence entre 450 et 100000 Da, de préférence entre 500 et 75000 Da, de préférence entre 550 et 50000 Da, de préférence entre 600 et 40000 Da, de préférence entre 650 et 30000 Da, de préférence entre 700 et 20000 Da, de préférence entre 750 et 10000 Da, de préférence entre 800 et 9000 Da, de préférence entre 850 et 8000 Da, de préférence entre 900 et 7000 Da, de préférence entre 950 et 6000 Da, de préférence entre 1000 et 5000 Da, de préférence entre 1050 et 4000 Da, de préférence entre 1100 et 3000 Da, de préférence entre 1150 et 2000 Da, de préférence entre 1200 et 1000 Da.

Avantageusement, selon l'invention, lesdits collagènes et/ou lesdits hydrolysats de collagène et/ou lesdites gélatines présentent un poids moléculaire compris entre 1000 et 300000 Da, de préférence entre 1500 et 150000 Da, de préférence entre 2000 et 60000 Da.

De préférence, selon l'invention, lesdits collagènes et/ou lesdits hydrolysats de collagène présentent un poids moléculaire égal à 50 Da ou égal à 100 Da ou égal à 150 Da ou égal à 200 Da ou égal à 250 Da ou égal à 300 Da ou égal à 350 Da ou égal à 400 Da ou égal à 450 Da ou égal à 500 Da ou égal à 550 Da ou égal à 600 Da ou égal à 650 Da ou égal à 700 Da ou égal à 750 Da ou égal à 800 Da ou égal à 850 Da ou égal à 900 ou égal à 950 Da ou égal à 1000 Da ou égal à 1100 Da ou égal à 1200 ou égal à 1300 Da ou égal à 1400 Da ou égal à 1500 Da ou égal à 1600 Da ou égal à 1700 Da ou égal à 1800 Da ou égal à 1900 Da ou égal à 2000 Da ou égal à 2500 Da ou égal à 3000 Da ou égal à 3500 Da ou égal à 4000 Da ou égal à 4500 Da ou égal à 5000 Da ou égal à 5500 Da ou égal à 6000 Da ou égal à 6500 Da ou égal à 7000 Da ou égal à 7500 Da ou égal à 8000 Da ou égal à 8500 Da ou égal à 9000 Da ou égal à 9500 Da ou égal à 10000 Da ou égal à 12500 Da ou égal à 15000 Da ou égal à 17500 Da ou égal à 20000 Da ou égal à 22500 Da ou égal à 25000 Da ou égal à 27500 Da ou égal à 30000 Da ou égal à 32500 Da ou égal à 35000 Da ou égal à 37500 Da ou égal à 40000 Da ou égal à 42500 Da ou égal à 45000 Da ou égal à 47500 Da ou égal à 50000 Da ou égal à 55000 Da ou égal à 60000 Da ou égal à 65000 Da ou égal à 70000 Da ou égal à 75000 Da ou égal à 80000 Da ou égal à 85000 Da ou égal à 90000 Da ou égal à 100000 Da ou égal à 110000 Da ou égal à 120000 Da ou égal à 130000 Da ou égal à 140000 Da ou égal à 150000 Da ou égal à 160000 Da ou égal à 170000 Da ou égal à 180000 Da ou égal à 190000 da ou égal à 200000 Da ou égal à 210000 Da ou égal à 220000 Da ou égal à 230000 Da ou égal à 240000 Da ou égal à 250000 Da ou égal à 260000 Da ou égal à 270000 Da ou égal à 280000 Da ou égal à 290000 Da ou égal à 300000 Da.

Suivant un mode de réalisation selon l'invention, lorsque ladite au moins une protéine naturelle ou synthétique est du collagène, ce dernier présente un poids moléculaire compris entre 900 et 7000 Da, de préférence un poids moléculaire compris entre 950 et 5000 Da, préférentiellement un poids moléculaire compris entre 1000 et 3000 Da.

Suivant un mode de réalisation selon l'invention, lorsque ladite au moins une protéine naturelle ou synthétique est du collagène, ce dernier présente un poids moléculaire égal à 2000 Da ou égal à 3000 Da ou égal à 5000 Da ou égal à 50000 Da.

Suivant un mode de réalisation selon l'invention, lorsque ladite au moins une protéine naturelle ou synthétique est de la gélatine, cette dernière présente un poids moléculaire compris entre 900 et 6000 Da, de préférence un poids moléculaire compris entre 950 et 5000 Da, préférentiellement un poids moléculaire compris entre 1000 et 3000 Da.

Suivant un mode de réalisation selon l'invention, lorsque ladite au moins une protéine naturelle ou synthétique est de la gélatine, cette dernière présente un poids moléculaire égal à 2000 Da ou égal à 3000 Da ou égal à 5000 Da ou égal à 50000 Da.

Suivant un mode de réalisation selon l'invention, lorsque ladite au moins une protéine naturelle ou synthétique est un collagène hydrolysé ou un peptide de collagène, ce dernier présente un poids moléculaire compris entre 900 et 6000 Da, de préférence un poids moléculaire compris entre 950 et 5000 Da, préférentiellement un poids moléculaire compris entre 1000 et 3000 Da.

Suivant un mode de réalisation selon l'invention, lorsque ladite au moins une protéine naturelle ou synthétique est un collagène hydrolysé ou un peptide de collagène, ce dernier présente un poids moléculaire égal à 2000 Da ou égal à 3000 Da ou égal à 5000 Da ou égal à 50000 Da.

Selon un mode de réalisation, la composition suivant l'invention comprend en outre au moins un support naturel ou synthétique additionnel, par exemple de l'acétate de polyvinyle, du polyvinylpyrrolidone (PVP), de l'acétate de polyvinylpyrrolidone-co-vinyle, de l'acétate de polyethylène-co-vinyle, de l'acétate co-méthacrylique d'acide polyvinyle, du polyethylène oxide, du polylactide-co-glycolide, de l'alcool polyvinylique, du polycarbophile, de la polycaprolactone, du copolymère d'éthylène-vinyle, leurs dérivés et leurs mélanges.

Avantageusement, la composition selon l'invention comprend en outre au moins un agent plastifiant. L'addition d'un agent plastifiant dans une composition selon l'invention permet d'obtenir une composition selon l'invention au travers d'un procédé de fabrication où des températures inférieures aux points de fusion dudit au moins un cannabinoïde non-euphorisant naturel ou synthétique et du support peuvent être utilisées afin de garantir tout de même une fonte de ces deux composés et la dispersion/répartition/distribution dudit au moins un cannabinoïde non-euphorisant naturel ou synthétique au sein du support.

Préférentiellement, selon l'invention, ledit agent plastifiant est choisi dans le groupe constitué des polyols, des lipides, des lécithines, des esters de sucrose, de l'eau, du citrate de triéthyle, du polyéthylène glycol, du glycérol, du sébate de dibutyle, du stéarate de butyle, du monostéarate de glycérol, du sodium dodécylsulphate, du phtalate diéthyle, de leurs dérivés et de leurs mélanges.

Selon l'invention, les agents plastifiants préférés sont le glycérol, l'eau, le polyéthylène glycol et le citrate de triéthyle.

Avantageusement, selon l'invention, ledit au moins un cannabinoïde non-euphorisant est présent à raison de 1% à 80% en poids par rapport au poids total de la composition, de préférence à raison de 10% à 60% en poids par rapport au poids total de la composition, de préférence à raison de 15% à 50% en poids par rapport au poids total de la composition, préférentiellement à raison de 20% à 40% en poids par rapport au poids total de la composition.

Préférentiellement, selon l'invention, ledit au moins un cannabinoïde non-euphorisant est présent à raison de 20% en poids par rapport au poids total de la composition, ou à raison de 25% en poids par rapport au poids total de la composition, ou à raison de 30% en poids par rapport au poids total de la composition, ou à raison de 35% en poids par rapport au poids total de la composition, ou à raison de 40% en poids par rapport au poids total de la composition.

De préférence, selon l'invention, ledit au moins un support naturel ou synthétique choisi dans le groupe constitué des acides aminés, des peptides et des polypeptides naturels ou synthétiques, des protéines naturelles ou synthétiques sélectionnées parmi sélectionnées parmi les glycoprotéines, les collagènes, les hydrolysats de collagènes, les gélatines, les protéines de pois, les protéines de soja, les protéines de blé, les protéines de citrouille, les protéines de noix, les protéines de riz, les protéines de noisette, les protéines de lait, les protéines de petit pois, les protéines de lentilles, les protéines d'amandes, les protéines de pistaches, les protéines de chia, les protéines de chanvre, les protéines de lin, les protéines de courge, les protéines de sésame, les protéines de pavot, les protéines de lupin, les protéines de tournesol, les protéines d'avoine, les protéines de millet, les protéines d'orge, les protéines de seigle, leurs dérivés et leurs mélanges, des saccharides naturels ou synthétiques sélectionnées parmi l'alginate de calcium, l'alginate de sodium, l'alginate d'ammonium, l'alginate de propylène glycol, les amidons, les amidons prégélatinisés, les amidons modifiés, les fibres solubles ou insolubles, les carraghénanes, la pectine, l'hydroxypropylcellulose (HPC), le glycogène, le β-glucane, l'amylopectine, l'amylose, les dextrines, les cyclodextrines, la maltodextrine, l'isomaltose, l'isomalt, le xylane, le pullulan, l'agar-agar, les mannanes, le fucoïdane, la gomme de xanthane, la gomme de guar, la gomme mastic, la gomme arabique, la gomme d'acacia, la gomme adragante, la gomme ghatti, la gomme de karaya, la gomme de Konjac, la gomme de fenugrec, la gomme d'acajou ou anacarde, la gomme tara, la gomme de caroube, la gomme de cassia, la gomme de benjoin, la gomme cassia, la gomme gaïac, le chitosan, la chitine, la lévane, la néoserine, l'acide hyaluronique, les hyaluronates, le sulfate de chondroïtine, le dermatane sulfate, le kératane sulfate, le raffinose, le rhamminose, le mannose, le glucose, le rhamnose, le saccharose, le stachyose, le verbascose, le tréhalose, le lactose, le lactulose, le maltose, l'érythritol, le mannitol, le sorbitol, le xylitol, leurs dérivés et leurs mélanges, des lipides naturels ou synthétiques sélectionnés parmi les cires d'abeille, la cire de candelilla, la cire de son de riz, la cire de carnauba, les cires microcristallines, les huiles animales ou végétales, les huiles animales ou végétales hydrogénées, les phospholipides, les céramides, les esters d'acide gras, leurs dérivés et leurs mélanges, de l'urée, de ses dérivés et leurs mélanges, est présent à raison de 5% à 90% en poids par rapport au poids total de la composition, de préférence à raison de 15% à 85% en poids par rapport au poids total de la composition, préférentiellement à raison de 20% à 80% en poids par rapport au poids total de la composition, plus préférentiellement à raison de 50% à 75% en poids par rapport au poids total de la composition.

Avantageusement, selon l'invention, ledit au moins un support naturel ou synthétique choisi dans le groupe constitué des acides aminés, des peptides et des polypeptides naturels ou synthétiques, des protéines naturelles ou synthétiques sélectionnées parmi sélectionnées parmi les glycoprotéines, les collagènes, les hydrolysats de collagènes, les gélatines, les protéines de pois, les protéines de soja, les protéines de blé, les protéines de citrouille, les protéines de noix, les protéines de riz, les protéines de noisette, les protéines de lait, les protéines de petit pois, les protéines de lentilles, les protéines d'amandes, les protéines de pistaches, les protéines de chia, les protéines de chanvre, les protéines de lin, les protéines de courge, les protéines de sésame, les protéines de pavot, les protéines de lupin, les protéines de tournesol, les protéines d'avoine, les protéines de millet, les protéines d'orge, les protéines de seigle, leurs dérivés et leurs mélanges, des saccharides naturels ou synthétiques sélectionnées parmi l'alginate de calcium, l'alginate de sodium, l'alginate d'ammonium, l'alginate de propylène glycol, les amidons, les amidons prégélatinisés, les amidons modifiés, les fibres solubles ou insolubles, les carraghénanes, la pectine, l'hydroxypropylcellulose (HPC), le glycogène, le β-glucane, l'amylopectine, l'amylose, les dextrines, les cyclodextrines, la maltodextrine, l'isomaltose, l'isomalt, le xylane, le pullulan, l'agar-agar, les mannanes, le fucoïdane, la gomme de xanthane, la gomme de guar, la gomme mastic, la gomme arabique, la gomme d'acacia, la gomme adragante, la gomme ghatti, la gomme de karaya, la gomme de Konjac, la gomme de fenugrec, la gomme d'acajou ou anacarde, la gomme tara, la gomme de caroube, la gomme de cassia, la gomme de benjoin, la gomme cassia, la gomme gaïac, le chitosan, la chitine, la lévane, la néoserine, l'acide hyaluronique, les hyaluronates, le sulfate de chondroïtine, le dermatane sulfate, le kératane sulfate, le raffinose, le rhamminose, le mannose, le glucose, le rhamnose, le saccharose, le stachyose, le verbascose, le tréhalose, le lactose, le lactulose, le maltose, l'érythritol, le mannitol, le sorbitol, le xylitol, leurs dérivés et leurs mélanges, des lipides naturels ou synthétiques sélectionnés parmi les cires d'abeille, la cire de candelilla, la cire de son de riz, la cire de carnauba, les cires microcristallines, les huiles animales ou végétales, les huiles animales ou végétales hydrogénées, les phospholipides, les céramides, les esters d'acide gras, leurs dérivés et leurs mélanges, de l'urée, de ses dérivés et leurs mélanges, est présent à raison de 5% en poids par rapport au poids total de la composition, ou à raison de 10% en poids par rapport au poids total de la composition , ou à raison de 15% en poids par rapport au poids total de la composition, ou à raison de 20% en poids par rapport au poids total de la composition, ou à raison de 25% en poids par rapport au poids total de la composition, ou à raison de 30% en poids par rapport au poids total de la composition, ou à raison de 35% en poids par rapport au poids total de la composition, ou à raison de 40% en poids par rapport au poids total de la composition, ou à raison de 45% en poids par rapport au poids total de la composition, ou à raison de 50% en poids par rapport au poids total de la composition, ou à raison de 55% en poids par rapport au poids total de la composition, ou à raison de 60% en poids par rapport au poids total de la composition, ou à raison de 65% en poids par rapport au poids total de la composition, ou à raison de 70% en poids par rapport au poids total de la composition, ou à raison de 75% en poids par rapport au poids total de la composition.

Lorsqu'il s'agit d'un mélange de deux supports selon l'invention, le ratio entre ces deux supports est préférentiellement de l'ordre de 10:90 ou de l'ordre de 20:80 ou de l'ordre de 30:70 ou de l'ordre de 40:60 ou de l'ordre de 50:50.

Avantageusement, selon l'invention, ledit au moins un agent plastifiant est présent à raison de 5% à 30% en poids par rapport au poids total de la composition, de préférence à raison de 10% à 25% en poids par rapport au poids total de la composition, préférentiellement à raison de 15% à 20% en poids par rapport au poids total de la composition.

Avantageusement, selon l'invention, ledit au moins un agent plastifiant est présent à raison de 5% en poids par rapport au poids total de la composition, ou à raison de 10% en poids par rapport au poids total de la composition, à raison de 15% en poids par rapport au poids total de la composition, à raison de 20% en poids par rapport au poids total de la composition, à raison de 25% en poids par rapport au poids total de la composition, à raison de 30% en poids par rapport au poids total de la composition.

De préférence, la composition selon l'invention comprend en outre au moins un additif choisi dans le groupe constitué des agents lubrifiants, des agents surfactants, des agents antioxydants, des agents chélatants, de leurs dérivés et de leurs mélanges.

A titre d'exemple, peuvent être utilisés, seuls ou en mélange, en tant qu'agents lubrifiants dans une composition selon l'invention les composés suivants : le dibéhénate de glycérol, le talc, la silice, l'acide stéarique, l'acide borique, les cires, l'oléate de sodium, l'acétate de sodium, le stéarate de magnésium, le stéarate de calcium, le stéarate de sodium, le benzoate de sodium, le laurylsulfate de sodium, le distéarate de glycérol, le palmitostéarate de glycérol, la cellulose microcristalline ou encore les polyoxyl-8-glycérides.

A titre d'exemple, peuvent être utilisés, seuls ou en mélange, en tant qu'agents surfactants dans une composition selon l'invention les composés suivants : le PIuronic^{®}, le Span^{®}, le Cremophor^{®}, les polysorbates (Tween^{®}, ...), la vitamine E TPGS et le ducosate de sodium.

A titre d'exemple, peuvent être utilisés, seuls ou en mélange, en tant qu'agents antioxydants et/ou agents chélatants dans une composition selon l'invention les composés suivants : l'hydroxytoluène butylé, l'hydroxyanisiole butylé, l'EDTA, l'acide citrique et la vitamine E.

Avantageusement, la composition selon l'invention comprend en outre au moins un composé additionnel de type polyphénol choisi dans le groupe constitué des acides phénoliques, des stilbènes, des alcools phénoliques, des lignanes, des flavonoïdes, de leurs dérivés et de leurs mélanges. En particulier, les formes glycosylée et aglycone des polyphénols sont envisagées en tant que principe actif additionnel selon la présente invention. Plus particulièrement, au sens de la présente invention, le terme « polyphénol » désigne tant les polyphénols d'origine naturelle que les polyphénols de synthèse mais aussi tous les dérivés de polyphénols.

A titre d'exemple, au sens de la présente invention, peuvent être cités en tant qu'acides phénoliques, les dérivés de l'acide hydroxybenzoïque (acide gallique, acide tannique, ...) et les dérivés de l'acide hydroxycinamique (curcumine, acide coumarique, acide caféique, acide férulique, ...).

A titre d'exemple, au sens de la présente invention, peuvent être cités en tant que stilbènes, le resvératrol, le sirtinol, le picéatannol ou encore la polydatine.

A titre d'exemple, au sens de la présente invention, peuvent être cités en tant que flavonoïdes, les flavanoles (quercétine, myricétine, kaempférol, isorhamnétine, morine, rutine, tiliroside, trihydroxyéthylrutine, fisétine, ...), les flavones (apigénine, lutéoline, baicaléine, chrysine, diosmine, nobilétine, tangérétine, wogonine, aminogénistéine, ...), les flavanones (bavachine, 8-isopenténylnaringénine, isoxanthohumole, naringénine, eriodictyole, hespérétine, silybine, taxifoline, ...), les isoflavones (génistéine, daidzéine, daidzine, formonétine, génistine, néobavaisoflavone, puéranine, ...), les antocianidines (cianidine, pelargonidine, delphinidine, pétunidine, malvidine, ...) et les flavanols (cathéchines, gallocatéchine, épigallocatéchinegallate, ...).

Selon l'invention, ledit au moins un principe actif additionnel de type polyphénol constitue un inhibiteur/modulateur des pompes à efflux dont la P-gp.

Préférentiellement, la composition selon l'invention comprend en outre au moins un inhibiteur et/ou un modulateur de l'activité de la P-gp.

De préférence, la composition selon l'invention est conditionnée sous forme de pellets, de flakes, de granulés, de poudres, de comprimés effervescents ou non, de solutions injectables ou non, de suspensions, de gels, de pommades ou encore sous toute autre forme adéquate permettant une administration à un animal ou à un être humain.

D'autres formes de réalisation d'une composition suivant l'invention sont indiquées dans les revendications annexées.

L'invention a aussi pour objet un procédé de fabrication, en particulier un procédé de fabrication par thermoformage, d'une composition sous forme d'un extrudat thermoformé friable / cassant selon l'invention, caractérisé en ce qu'il comprend les étapes suivantes :
a) une étape d'amenée simultanée ou différée dans le temps d'au moins un cannabinoïde non-euphorisant naturel ou synthétique et d'au moins un support choisi dans le groupe constitué des acides aminés, des peptides et des polypeptides naturels ou synthétiques, des protéines naturelles ou synthétiques sélectionnées parmi les glycoprotéines, les collagènes, les hydrolysats de collagènes, les gélatines, les protéines de pois, les protéines de soja, les protéines de blé, les protéines de citrouille, les protéines de noix, les protéines de riz, les protéines de noisette, les protéines de lait, les protéines de petit pois, les protéines de lentilles, les protéines d'amandes, les protéines de pistaches, les protéines de chia, les protéines de chanvre, les protéines de lin, les protéines de courge, les protéines de sésame, les protéines de pavot, les protéines de lupin, les protéines de tournesol, les protéines d'avoine, les protéines de millet, les protéines d'orge, les protéines de seigle, leurs dérivés et leurs mélanges, des saccharides naturels ou synthétiques sélectionnés parmi l'alginate de calcium, l'alginate de sodium, l'alginate d'ammonium, l'alginate de propylène glycol, les amidons, les amidons prégélatinisés, les amidons modifiés, les fibres solubles ou insolubles, les carraghénanes, la pectine, l'hydroxypropylcellulose (HPC), le glycogène, le β-glucane, l'amylopectine, l'amylose, les dextrines, les cyclodextrines, la maltodextrine, l'isomaltose, l'isomalt, le xylane, le pullulan, l'agar-agar, les mannanes, le fucoïdane, la gomme de xanthane, la gomme de guar, la gomme mastic, la gomme arabique, la gomme d'acacia, la gomme adragante, la gomme ghatti, la gomme de karaya, la gomme de Konjac, la gomme de fenugrec, la gomme d'acajou ou anacarde, la gomme tara, la gomme de caroube, la gomme de cassia, la gomme de benjoin, la gomme cassia, la gomme gaïac, le chitosan, la chitine, la lévane, la néoserine, l'acide hyaluronique, les hyaluronates, le sulfate de chondroïtine, le dermatane sulfate, le kératane sulfate, le raffinose, le rhamminose, le mannose, le glucose, le rhamnose, le saccharose, le stachyose, le verbascose, le tréhalose, le lactose, le lactulose, le maltose, l'érythritol, le mannitol, le sorbitol, le xylitol, leurs dérivés et leurs mélanges, des lipides naturels ou synthétiques sélectionnés parmi les cires d'abeille, la cire de candelilla, la cire de son de riz, la cire de carnauba, les cires microcristallines, les huiles animales ou végétales, les huiles animales ou végétales hydrogénées, les phospholipides, les céramides, les esters d'acide gras, leurs dérivés et leurs mélanges, de l'urée, de ses dérivés et leurs mélanges, pour alimenter un extrudeur,
b) une étape de mélange, dans ledit extrudeur, dudit au moins un cannabinoïde non-euphorisant naturel ou synthétique et dudit au moins un support choisi dans le groupe constitué des acides aminés, des peptides et des polypeptides naturels ou synthétiques, des protéines naturelles ou synthétiques sélectionnées parmi les glycoprotéines, les collagènes, les hydrolysats de collagènes, les gélatines, les protéines de pois, les protéines de soja, les protéines de blé, les protéines de citrouille, les protéines de noix, les protéines de riz, les protéines de noisette, les protéines de lait, les protéines de petit pois, les protéines de lentilles, les protéines d'amandes, les protéines de pistaches, les protéines de chia, les protéines de chanvre, les protéines de lin, les protéines de courge, les protéines de sésame, les protéines de pavot, les protéines de lupin, les protéines de tournesol, les protéines d'avoine, les protéines de millet, les protéines d'orge, les protéines de seigle, leurs dérivés et leurs mélanges, des saccharides naturels ou synthétiques sélectionnés parmi l'alginate de calcium, l'alginate de sodium, l'alginate d'ammonium, l'alginate de propylène glycol, les amidons, les amidons prégélatinisés, les amidons modifiés, les fibres solubles ou insolubles, les carraghénanes, la pectine, l'hydroxypropylcellulose (HPC), le glycogène, le β-glucane, l'amylopectine, l'amylose, les dextrines, les cyclodextrines, la maltodextrine, l'isomaltose, l'isomalt, le xylane, le pullulan, l'agar-agar, les mannanes, le fucoïdane, la gomme de xanthane, la gomme de guar, la gomme mastic, la gomme arabique, la gomme d'acacia, la gomme adragante, la gomme ghatti, la gomme de karaya, la gomme de Konjac, la gomme de fenugrec, la gomme d'acajou ou anacarde, la gomme tara, la gomme de caroube, la gomme de cassia, la gomme de benjoin, la gomme cassia, la gomme gaïac, le chitosan, la chitine, la lévane, la néoserine, l'acide hyaluronique, les hyaluronates, le sulfate de chondroïtine, le dermatane sulfate, le kératane sulfate, le raffinose, le rhamminose, le mannose, le glucose, le rhamnose, le saccharose, le stachyose, le verbascose, le tréhalose, le lactose, le lactulose, le maltose, l'érythritol, le mannitol, le sorbitol, le xylitol, leurs dérivés et leurs mélanges, des lipides naturels ou synthétiques sélectionnés parmi les cires d'abeille, la cire de candelilla, la cire de son de riz, la cire de carnauba, les cires microcristallines, les huiles animales ou végétales, les huiles animales ou végétales hydrogénées, les phospholipides, les céramides, les esters d'acide gras, leurs dérivés et leurs mélanges, de l'urée, de ses dérivés et leurs mélanges, pour former un mélange, et
c) une étape d'extrusion à chaud dudit mélange obtenu à l'étape b) dans ledit extrudeur pour obtenir un extrudat thermoformé friable / cassant, en particulier pour obtenir un extrudat thermoformé friable / cassant consistant en une dispersion solide, plus particulièrement pour obtenir un extrudat thermoformé consistant en une dispersion solide consistant en une structure vitreuse comprenant un mélange moléculaire dudit au moins un cannabinoïde non-euphorisant et dudit au moins un support.

Un tel procédé selon l'invention donne lieu à une composition sous forme d'un extrudat thermoformé friable / cassant, c'est-à-dire obtenu par thermoformage et plus particulièrement par extrusion à chaud, dans lequel ledit au moins un cannabinoïde non-euphorisant naturel ou synthétique comme principe actif comprend préférentiellement au moins une première phase amorphe et éventuellement une deuxième phase cristalline dispersée(s) au sein dudit au moins un support choisi dans le groupe constitué des acides aminés, des peptides et des polypeptides naturels ou synthétiques, des protéines naturelles ou synthétiques sélectionnées parmi les glycoprotéines, les collagènes, les hydrolysats de collagènes, les gélatines, les protéines de pois, les protéines de soja, les protéines de blé, les protéines de citrouille, les protéines de noix, les protéines de riz, les protéines de noisette, les protéines de lait, les protéines de petit pois, les protéines de lentilles, les protéines d'amandes, les protéines de pistaches, les protéines de chia, les protéines de chanvre, les protéines de lin, les protéines de courge, les protéines de sésame, les protéines de pavot, les protéines de lupin, les protéines de tournesol, les protéines d'avoine, les protéines de millet, les protéines d'orge, les protéines de seigle, leurs dérivés et leurs mélanges, des saccharides naturels ou synthétiques sélectionnés parmi l'alginate de calcium, l'alginate de sodium, l'alginate d'ammonium, l'alginate de propylène glycol, les amidons, les amidons prégélatinisés, les amidons modifiés, les fibres solubles ou insolubles, les carraghénanes, la pectine, l'hydroxypropylcellulose (HPC), le glycogène, le β-glucane, l'amylopectine, l'amylose, les dextrines, les cyclodextrines, la maltodextrine, l'isomaltose, l'isomalt, le xylane, le pullulan, l'agar-agar, les mannanes, le fucoïdane, la gomme de xanthane, la gomme de guar, la gomme mastic, la gomme arabique, la gomme d'acacia, la gomme adragante, la gomme ghatti, la gomme de karaya, la gomme de Konjac, la gomme de fenugrec, la gomme d'acajou ou anacarde, la gomme tara, la gomme de caroube, la gomme de cassia, la gomme de benjoin, la gomme cassia, la gomme gaïac, le chitosan, la chitine, la lévane, la néoserine, l'acide hyaluronique, les hyaluronates, le sulfate de chondroïtine, le dermatane sulfate, le kératane sulfate, le raffinose, le rhamminose, le mannose, le glucose, le rhamnose, le saccharose, le stachyose, le verbascose, le tréhalose, le lactose, le lactulose, le maltose, l'érythritol, le mannitol, le sorbitol, le xylitol, leurs dérivés et leurs mélanges, des lipides naturels ou synthétiques sélectionnés parmi les cires d'abeille, la cire de candelilla, la cire de son de riz, la cire de carnauba, les cires microcristallines, les huiles animales ou végétales, les huiles animales ou végétales hydrogénées, les phospholipides, les céramides, les esters d'acide gras, leurs dérivés et leurs mélanges, de l'urée, de ses dérivés et leurs mélanges.

Cette composition suivant l'invention présente une solubilité et/ou une dispersion en phase aqueuse (milieu aqueux) nettement supérieure dudit au moins un cannabinoïde non-euphorisant naturel ou synthétique et simultanément une biodisponibilité significativement augmentée dudit au moins un cannabinoïde non-euphorisant naturel ou synthétique par rapport aux solubilités et/ou aux dispersions et aux biodisponibilités de ces composés pour les compositions actuelles. Il a été montré que la composition selon l'invention se présente sous forme d'un extrudat thermoformé friable / cassant dans lequel ledit au moins un cannabinoïde non-euphorisant naturel ou synthétique (principe actif) comprenant préférentiellement au moins une première phase amorphe et éventuellement une deuxième phase cristalline est dispersé au sein dudit au moins un support choisi dans le groupe constitué des acides aminés, des peptides et des polypeptides naturels ou synthétiques, des protéines naturelles ou synthétiques sélectionnées parmi les glycoprotéines, les collagènes, les hydrolysats de collagènes, les gélatines, les protéines de pois, les protéines de soja, les protéines de blé, les protéines de citrouille, les protéines de noix, les protéines de riz, les protéines de noisette, les protéines de lait, les protéines de petit pois, les protéines de lentilles, les protéines d'amandes, les protéines de pistaches, les protéines de chia, les protéines de chanvre, les protéines de lin, les protéines de courge, les protéines de sésame, les protéines de pavot, les protéines de lupin, les protéines de tournesol, les protéines d'avoine, les protéines de millet, les protéines d'orge, les protéines de seigle, leurs dérivés et leurs mélanges, des saccharides naturels ou synthétiques sélectionnées parmi l'alginate de calcium, l'alginate de sodium, l'alginate d'ammonium, l'alginate de propylène glycol, les amidons, les amidons prégélatinisés, les amidons modifiés, les fibres solubles ou insolubles, les carraghénanes, la pectine, l'hydroxypropylcellulose (HPC), le glycogène, le β-glucane, l'amylopectine, l'amylose, les dextrines, les cyclodextrines, la maltodextrine, l'isomaltose, l'isomalt, le xylane, le pullulan, l'agar-agar, les mannanes, le fucoïdane, la gomme de xanthane, la gomme de guar, la gomme mastic, la gomme arabique, la gomme d'acacia, la gomme adragante, la gomme ghatti, la gomme de karaya, la gomme de Konjac, la gomme de fenugrec, la gomme d'acajou ou anacarde, la gomme tara, la gomme de caroube, la gomme de cassia, la gomme de benjoin, la gomme cassia, la gomme gaïac, le chitosan, la chitine, la lévane, la néoserine, l'acide hyaluronique, les hyaluronates, le sulfate de chondroïtine, le dermatane sulfate, le kératane sulfate, le raffinose, le rhamminose, le mannose, le glucose, le rhamnose, le saccharose, le stachyose, le verbascose, le tréhalose, le lactose, le lactulose, le maltose, l'érythritol, le mannitol, le sorbitol, le xylitol, leurs dérivés et leurs mélanges, des lipides naturels ou synthétiques sélectionnés parmi les cires d'abeille, la cire de candelilla, la cire de son de riz, la cire de carnauba, les cires microcristallines, les huiles animales ou végétales, les huiles animales ou végétales hydrogénées, les phospholipides, les céramides, les esters d'acide gras, leurs dérivés et leurs mélanges, de l'urée, de ses dérivés et leurs mélanges.

Il a également été montré, dans le cadre de la présente invention, que ledit au moins un cannabinoïde non-euphorisant naturel ou synthétique n'est pas dégradé même lors du procédé de fabrication par thermoformage de la composition sous forme d'un extrudat thermoformé friable / cassant qui implique pourtant la soumission dudit au moins un cannabinoïde non-euphorisant naturel ou synthétique à de hautes températures (HME). Par ailleurs, il a également été mis en avant que ledit au moins un cannabinoïde non-euphorisant naturel ou synthétique dans une composition sous forme d'un extrudat thermoformé friable / cassant suivant l'invention y est réparti (distribué) de façon homogène.

Plus particulièrement, l'extrusion à chaud (Hot Melt Extrusion - HME) réalisée selon le procédé de fabrication par thermoformage suivant l'invention donne lieu à une fonte du principe actif (ledit au moins un cannabinoïde non-euphorisant naturel ou synthétique) et/ou dudit au moins un support à une température supérieure ou égale à leur point de fusion.

Toutefois, selon certains modes de réalisation d'une composition selon l'invention, cette fonte du principe actif et du support peut avoir lieu à une température inférieure à leur point de fusion. Ceci est par exemple le cas si la composition selon l'invention comprend un agent plastifiant ou si le principe actif lui-même présente des propriétés plastifiantes. Une telle fonte du principe actif et/ou du support donne lieu à une dispersion solide dans laquelle le principe actif (ledit au moins un cannabinoïde non-euphorisant naturel ou synthétique) comprenant préférentiellement au moins une première phase amorphe et éventuellement une deuxième phase cristalline est dispersé/réparti/distribué de façon homogène au sein dudit au moins un support choisi dans le groupe constitué des acides aminés, des peptides et des polypeptides naturels ou synthétiques, des protéines naturelles ou synthétiques sélectionnées parmi les glycoprotéines, les collagènes, les hydrolysats de collagènes, les gélatines, les protéines de pois, les protéines de soja, les protéines de blé, les protéines de citrouille, les protéines de noix, les protéines de riz, les protéines de noisette, les protéines de lait, les protéines de petit pois, les protéines de lentilles, les protéines d'amandes, les protéines de pistaches, les protéines de chia, les protéines de chanvre, les protéines de lin, les protéines de courge, les protéines de sésame, les protéines de pavot, les protéines de lupin, les protéines de tournesol, les protéines d'avoine, les protéines de millet, les protéines d'orge, les protéines de seigle, leurs dérivés et leurs mélanges, des saccharides naturels ou synthétiques sélectionnés parmi l'alginate de calcium, l'alginate de sodium, l'alginate d'ammonium, l'alginate de propylène glycol, les amidons, les amidons prégélatinisés, les amidons modifiés, les fibres solubles ou insolubles, les carraghénanes, la pectine, l'hydroxypropylcellulose (HPC), le glycogène, le β-glucane, l'amylopectine, l'amylose, les dextrines, les cyclodextrines, la maltodextrine, l'isomaltose, l'isomalt, le xylane, le pullulan, l'agar-agar, les mannanes, le fucoïdane, la gomme de xanthane, la gomme de guar, la gomme mastic, la gomme arabique, la gomme d'acacia, la gomme adragante, la gomme ghatti, la gomme de karaya, la gomme de Konjac, la gomme de fenugrec, la gomme d'acajou ou anacarde, la gomme tara, la gomme de caroube, la gomme de cassia, la gomme de benjoin, la gomme cassia, la gomme gaïac, le chitosan, la chitine, la lévane, la néoserine, l'acide hyaluronique, les hyaluronates, le sulfate de chondroïtine, le dermatane sulfate, le kératane sulfate, le raffinose, le rhamminose, le mannose, le glucose, le rhamnose, le saccharose, le stachyose, le verbascose, le tréhalose, le lactose, le lactulose, le maltose, l'érythritol, le mannitol, le sorbitol, le xylitol, leurs dérivés et leurs mélanges, des lipides naturels ou synthétiques sélectionnés parmi les cires d'abeille, la cire de candelilla, la cire de son de riz, la cire de carnauba, les cires microcristallines, les huiles animales ou végétales, les huiles animales ou végétales hydrogénées, les phospholipides, les céramides, les esters d'acide gras, leurs dérivés et leurs mélanges, de l'urée, de ses dérivés et leurs mélanges.

Avantageusement, selon le procédé suivant l'invention, l'étape c) d'extrusion à chaud est une étape selon laquelle ledit cannabinoïde non-euphorisant et/ou ledit au moins un support choisi dans le groupe constitué des acides aminés, des peptides et des polypeptides naturels ou synthétiques, des protéines naturelles ou synthétiques sélectionnées parmi les glycoprotéines, les collagènes, les hydrolysats de collagènes, les gélatines, les protéines de pois, les protéines de soja, les protéines de blé, les protéines de citrouille, les protéines de noix, les protéines de riz, les protéines de noisette, les protéines de lait, les protéines de petit pois, les protéines de lentilles, les protéines d'amandes, les protéines de pistaches, les protéines de chia, les protéines de chanvre, les protéines de lin, les protéines de courge, les protéines de sésame, les protéines de pavot, les protéines de lupin, les protéines de tournesol, les protéines d'avoine, les protéines de millet, les protéines d'orge, les protéines de seigle, leurs dérivés et leurs mélanges, des saccharides naturels ou synthétiques sélectionnés parmi l'alginate de calcium, l'alginate de sodium, l'alginate d'ammonium, l'alginate de propylène glycol, les amidons, les amidons prégélatinisés, les amidons modifiés, les fibres solubles ou insolubles, les carraghénanes, la pectine, l'hydroxypropylcellulose (HPC), le glycogène, le β-glucane, l'amylopectine, l'amylose, les dextrines, les cyclodextrines, la maltodextrine, l'isomaltose, l'isomalt, le xylane, le pullulan, l'agar-agar, les mannanes, le fucoïdane, la gomme de xanthane, la gomme de guar, la gomme mastic, la gomme arabique, la gomme d'acacia, la gomme adragante, la gomme ghatti, la gomme de karaya, la gomme de Konjac, la gomme de fenugrec, la gomme d'acajou ou anacarde, la gomme tara, la gomme de caroube, la gomme de cassia, la gomme de benjoin, la gomme cassia, la gomme gaïac, le chitosan, la chitine, la lévane, la néoserine, l'acide hyaluronique, les hyaluronates, le sulfate de chondroïtine, le dermatane sulfate, le kératane sulfate, le raffinose, le rhamminose, le mannose, le glucose, le rhamnose, le saccharose, le stachyose, le verbascose, le tréhalose, le lactose, le lactulose, le maltose, l'érythritol, le mannitol, le sorbitol, le xylitol, leurs dérivés et leurs mélanges, des lipides naturels ou synthétiques sélectionnés parmi les cires d'abeille, la cire de candelilla, la cire de son de riz, la cire de carnauba, les cires microcristallines, les huiles animales ou végétales, les huiles animales ou végétales hydrogénées, phospholipides, les céramides, les esters d'acide gras, leurs dérivés et leurs mélanges, de l'urée, de ses dérivés et leurs mélanges, subisse/subissent un changement d'état de la matière, ledit au moins un cannabinoïde non-euphorisant et/ou ledit au moins un support passant d'un premier état de la matière à un second état de la matière, lequel second état de la matière impliquant en particulier que ledit au moins un cannabinoïde non-euphorisant et/ou ledit au moins un support se présente/présentent au moins partiellement sous forme amorphe.

Avantageusement, le procédé suivant l'invention comprend une étape préalable de pré-mélange dudit au moins un cannabinoïde non-euphorisant naturel ou synthétique et dudit au moins un support choisi dans le groupe constitué des acides aminés, des peptides et des polypeptides naturels ou synthétiques, des protéines naturelles ou synthétiques sélectionnées parmi les glycoprotéines, les collagènes, les hydrolysats de collagènes, les gélatines, les protéines de pois, les protéines de soja, les protéines de blé, les protéines de citrouille, les protéines de noix, les protéines de riz, les protéines de noisette, les protéines de lait, les protéines de petit pois, les protéines de lentilles, les protéines d'amandes, les protéines de pistaches, les protéines de chia, les protéines de chanvre, les protéines de lin, les protéines de courge, les protéines de sésame, les protéines de pavot, les protéines de lupin, les protéines de tournesol, les protéines d'avoine, les protéines de millet, les protéines d'orge, les protéines de seigle, leurs dérivés et leurs mélanges, des saccharides naturels ou synthétiques sélectionnés parmi l'alginate de calcium, l'alginate de sodium, l'alginate d'ammonium, l'alginate de propylène glycol, les amidons, les amidons prégélatinisés, les amidons modifiés, les fibres solubles ou insolubles, les carraghénanes, la pectine, l'hydroxypropylcellulose (HPC), le glycogène, le β-glucane, l'amylopectine, l'amylose, les dextrines, les cyclodextrines, la maltodextrine, l'isomaltose, l'isomalt, le xylane, le pullulan, l'agar-agar, les mannanes, le fucoïdane, la gomme de xanthane, la gomme de guar, la gomme mastic, la gomme arabique, la gomme d'acacia, la gomme adragante, la gomme ghatti, la gomme de karaya, la gomme de Konjac, la gomme de fenugrec, la gomme d'acajou ou anacarde, la gomme tara, la gomme de caroube, la gomme de cassia, la gomme de benjoin, la gomme cassia, la gomme gaïac, le chitosan, la chitine, la lévane, la néoserine, l'acide hyaluronique, les hyaluronates, le sulfate de chondroïtine, le dermatane sulfate, le kératane sulfate, le raffinose, le rhamminose, le mannose, le glucose, le rhamnose, le saccharose, le stachyose, le verbascose, le tréhalose, le lactose, le lactulose, le maltose, l'érythritol, le mannitol, le sorbitol, le xylitol, leurs dérivés et leurs mélanges, des lipides naturels ou synthétiques sélectionnés parmi les cires d'abeille, la cire de candelilla, la cire de son de riz, la cire de carnauba, les cires microcristallines, les huiles animales ou végétales, les huiles animales ou végétales hydrogénées, les phospholipides, les céramides, les esters d'acide gras, leurs dérivés et leurs mélanges, de l'urée, de ses dérivés et leurs mélanges, de telle sorte à former un pré-mélange destiné à alimenter l'extrudeur.

De façon préférée, selon le procédé suivant l'invention, ladite étape d'extrusion à chaud est réalisée à une température d'extrusion ou température de thermoformage comprise entre 20 et 180°C, de préférence à une température comprise entre 40 et 115°C, préférentiellement à une température comprise entre 80 et 90°C, de préférence à une température égale à 115°C, de préférence à une température égale à 110°C, de préférence à une température égale à 100°C, de préférence à une température égale à 90°C, de préférence à une température égale à 80°C.

Avantageusement, selon le procédé suivant l'invention, ladite étape d'extrusion à chaud est réalisée à une vitesse de rotation d'une vis d'extrusion comprise entre 20 et 900 tours/min, de préférence comprise entre 50 et 300 tours/min, de préférence comprise entre 100 et 250 tours/min, préférentiellement égale à 250 tours/min, préférentiellement égale à 200 tours/min.

Préférentiellement, le procédé selon l'invention comprend une étape additionnelle de refroidissement en sortie de l'extrudeur.

Avantageusement, le procédé selon l'invention comprend une étape additionnelle de traitement de l'extrudat thermoformé friable / cassant en sortie de l'extrudeur, par exemple une découpe au niveau d'un pelletiseur et/ou un broyage et/ou un concassage et/ou une réduction en poudre (sous forme de poudre) dudit extrudat thermoformé friable / cassant.

D'autres formes de réalisation du procédé suivant l'invention sont indiquées dans les revendications annexées.

La présente invention porte également sur une composition sous forme d'un extrudat thermoformé friable / cassant, éventuellement conditionné en pellet, en flake, en granulé, en poudre, en comprimé effervescent ou non, en solution injectable ou non, en suspension, en gel, en pommade ou encore sous toute autre forme adéquate permettant une administration à un animal ou à un être humain, obtenu suivant le procédé selon l'invention, ladite composition comprenant au moins un cannabinoïde non-euphorisant naturel ou synthétique comme principe actif et au moins un support choisi dans le groupe constitué des acides aminés, des peptides et des polypeptides naturels ou synthétiques, des protéines naturelles ou synthétiques sélectionnées parmi les glycoprotéines, les collagènes, les hydrolysats de collagènes, les gélatines, les protéines de pois, les protéines de soja, les protéines de blé, les protéines de citrouille, les protéines de noix, les protéines de riz, les protéines de noisette, les protéines de lait, les protéines de petit pois, les protéines de lentilles, les protéines d'amandes, les protéines de pistaches, les protéines de chia, les protéines de chanvre, les protéines de lin, les protéines de courge, les protéines de sésame, les protéines de pavot, les protéines de lupin, les protéines de tournesol, les protéines d'avoine, les protéines de millet, les protéines d'orge, les protéines de seigle, leurs dérivés et leurs mélanges, des saccharides naturels ou synthétiques sélectionnés parmi l'alginate de calcium, l'alginate de sodium, l'alginate d'ammonium, l'alginate de propylène glycol, les amidons, les amidons prégélatinisés, les amidons modifiés, les fibres solubles ou insolubles, les carraghénanes, la pectine, l'hydroxypropylcellulose (HPC), le glycogène, le β-glucane, l'amylopectine, l'amylose, les dextrines, les cyclodextrines, la maltodextrine, l'isomaltose, l'isomalt, le xylane, le pullulan, l'agar-agar, les mannanes, le fucoïdane, la gomme de xanthane, la gomme de guar, la gomme mastic, la gomme arabique, la gomme d'acacia, la gomme adragante, la gomme ghatti, la gomme de karaya, la gomme de Konjac, la gomme de fenugrec, la gomme d'acajou ou anacarde, la gomme tara, la gomme de caroube, la gomme de cassia, la gomme de benjoin, la gomme cassia, la gomme gaïac, le chitosan, la chitine, la lévane, la néoserine, l'acide hyaluronique, les hyaluronates, le sulfate de chondroïtine, le dermatane sulfate, le kératane sulfate, le raffinose, le rhamminose, le mannose, le glucose, le rhamnose, le saccharose, le stachyose, le verbascose, le tréhalose, le lactose, le lactulose, le maltose, l'érythritol, le mannitol, le sorbitol, le xylitol, leurs dérivés et leurs mélanges, des lipides naturels ou synthétiques sélectionnés parmi les cires d'abeille, la cire de candelilla, la cire de son de riz, la cire de carnauba, les cires microcristallines, les huiles animales ou végétales, les huiles animales ou végétales hydrogénées, les phospholipides, les céramides, les esters d'acide gras, leurs dérivés et leurs mélanges, de l'urée, de ses dérivés et leurs mélanges, ledit au moins un cannabinoïde non-euphorisant naturel ou synthétique comprenant préférentiellement au moins une première phase amorphe et éventuellement une deuxième phase cristalline.

En d'autres termes, la présente invention porte également sur une composition sous forme d'un extrudat thermoformé friable / cassant, éventuellement conditionné en pellet, en flake, en granulé, en poudre, en comprimé effervescent ou non, en solution injectable ou non, en suspension, en gel, en pommade ou encore sous toute autre forme adéquate permettant une administration à un animal ou à un être humain, obtenu par extrusion à chaud (HME - Hot Melt Extrusion), ladite composition comprenant au moins un cannabinoïde non-euphorisant naturel ou synthétique comme principe actif et au moins un support choisi dans le groupe constitué des acides aminés, des peptides et des polypeptides naturels ou synthétiques, des protéines naturelles ou synthétiques sélectionnées parmi les glycoprotéines, les collagènes, les hydrolysats de collagènes, les gélatines, les protéines de pois, les protéines de soja, les protéines de blé, les protéines de citrouille, les protéines de noix, les protéines de riz, les protéines de noisette, les protéines de lait, les protéines de petit pois, les protéines de lentilles, les protéines d'amandes, les protéines de pistaches, les protéines de chia, les protéines de chanvre, les protéines de lin, les protéines de courge, les protéines de sésame, les protéines de pavot, les protéines de lupin, les protéines de tournesol, les protéines d'avoine, les protéines de millet, les protéines d'orge, les protéines de seigle, leurs dérivés et leurs mélanges, des saccharides naturels ou synthétiques sélectionnés parmi l'alginate de calcium, l'alginate de sodium, l'alginate d'ammonium, l'alginate de propylène glycol, les amidons, les amidons prégélatinisés, les amidons modifiés, les fibres solubles ou insolubles, les carraghénanes, la pectine, l'hydroxypropylcellulose (HPC), le glycogène, le β-glucane, l'amylopectine, l'amylose, les dextrines, les cyclodextrines, la maltodextrine, l'isomaltose, l'isomalt, le xylane, le pullulan, l'agar-agar, les mannanes, le fucoïdane, la gomme de xanthane, la gomme de guar, la gomme mastic, la gomme arabique, la gomme d'acacia, la gomme adragante, la gomme ghatti, la gomme de karaya, la gomme de Konjac, la gomme de fenugrec, la gomme d'acajou ou anacarde, la gomme tara, la gomme de caroube, la gomme de cassia, la gomme de benjoin, la gomme cassia, la gomme gaïac, le chitosan, la chitine, la lévane, la néoserine, l'acide hyaluronique, les hyaluronates, le sulfate de chondroïtine, le dermatane sulfate, le kératane sulfate, le raffinose, le rhamminose, le mannose, le glucose, le rhamnose, le saccharose, le stachyose, le verbascose, le tréhalose, le lactose, le lactulose, le maltose, l'érythritol, le mannitol, le sorbitol, le xylitol, leurs dérivés et leurs mélanges, des lipides naturels ou synthétiques sélectionnés parmi les cires d'abeille, la cire de candelilla, la cire de son de riz, la cire de carnauba, les cires microcristallines, les huiles animales ou végétales, les huiles animales ou végétales hydrogénées, les phospholipides, les céramides, les esters d'acide gras, leurs dérivés et leurs mélanges, de l'urée, de ses dérivés et leurs mélanges, ledit au moins un cannabinoïde non-euphorisant naturel ou synthétique comprenant préférentiellement au moins une première phase amorphe et éventuellement une deuxième phase cristalline.

La présente invention porte également sur une utilisation d'une composition suivant l'invention en tant que complément alimentaire et/ou en tant que produit cosmétique et/ou en tant que médicament à usage humain ou vétérinaire.

En particulier, la présente invention porte sur une composition pour utilisation dans le traitement préventif et/ou curatif, chez l'être humain et/ou chez l'animal, de pathologies en lien avec le système cardiovasculaire (ischémie, désordres métaboliques,...), de pathologies en lien avec le système gastro-intestinal (nausées, vomissements, diarrhées, inflammations digestives, ...), de pathologies en lien avec le vieillissement prématuré des cellules, de pathologies en lien avec le système endocrinien (hyperglycémie, ...), de pathologies en lien avec le système immunitaire (maladies inflammatoires, arthrite rhumatoïde, sclérose en plaque, ostéoarthrite, fibromyalgie,...), de pathologies en lien avec le système nerveux central (douleur, migraine, épilepsie, maladie de Parkinson, maladie d'Alzheimer, anxiété, dépression,...), des troubles du sommeil, de maladies de la peau, de maladies dues à la présence de microorganismes, des cancers (anti-tumoral, ...), des maladies oculaires (glaucoma,...), des maladies du système respiratoire (asthme,...) et dans le traitement préventif et/ou curatif du diabète.

Plus particulièrement, la présente invention porte sur une composition pour utilisation dans le traitement préventif et/ou curatif, chez l'être humain et/ou chez l'animal, des maladies liées aux désordres neuropsychiatriques, de la douleur, des maladies neurodégénératives, des maladies inflammatoires, des dermatites et des maladies de la peau ou encore du côlon irritable (IBS).

Plus particulièrement, la présente invention porte sur une composition utilisée, chez l'être humain et/ou chez l'animal, en tant qu'antioxydant, qu'anti-inflammatoire, qu'anticonvulsif, qu'antiémétique, qu'anxiolytique, qu'hypnotique ou encore en tant qu'antipsychotique.

Une composition selon l'invention présente préférentiellement des propriétés anti-inflammatoires, neuroprotectrices, anticonvulsivantes, anxyolitiques, antidepressions, antipsychotiques, antithrombotiques, antitumorales, antidiabétiques et/ou immunorégulatrices.

D'autres formes d'utilisation d'une composition suivant l'invention sont indiquées dans les revendications annexées.

D'autres caractéristiques, détails et avantages de l'invention ressortiront des exemples donnés ci-après, à titre non limitatif et en faisant référence aux figures annexées.

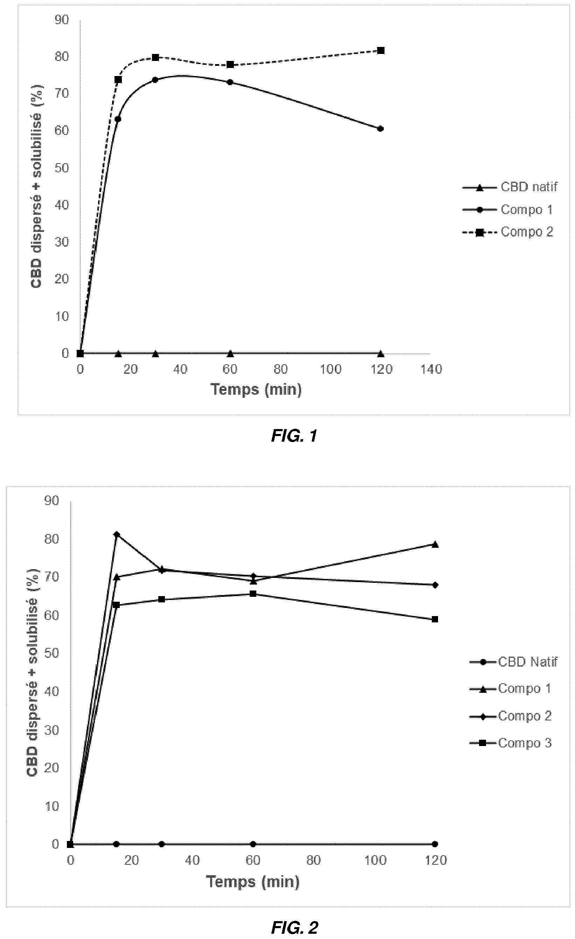

Les figures 1 à 3 sont des graphiques illustrant le taux de dispersion et de solubilisation d'un cannabinoïde non-euphorisant, en l'occurrence le taux de dispersion et de solubilisation du cannabidiol au cours du temps, au départ d'exemples de compositions selon l'invention.

### Exemples

### Exemple 1 : procédé de fabrication par thermoformage de compositions selon l'invention sous forme d'extrudats thermoformés friable / cassant

Des compositions thermoformées selon l'invention comprenant au moins un cannabinoïde non-euphorisant, telles que celles faisant l'objet de l'exemple 2 ci-dessous, ont été obtenues selon le procédé suivant faisant également l'objet de la présente invention :
a) une étape de pré-mélange d'au moins un cannabinoïde non-euphorisant à l'état cristallin sous forme de poudre et d'au moins un support choisi dans le groupe constitué des acides aminés, des peptides et des polypeptides naturels ou synthétiques, des protéines naturelles ou synthétiques sélectionnées parmi les glycoprotéines, les collagènes, les hydrolysats de collagènes, les gélatines, les protéines de pois, les protéines de soja, les protéines de blé, les protéines de citrouille, les protéines de noix, les protéines de riz, les protéines de noisette, les protéines de lait, les protéines de petit pois, les protéines de lentilles, les protéines d'amandes, les protéines de pistaches, les protéines de chia, les protéines de chanvre, les protéines de lin, les protéines de courge, les protéines de sésame, les protéines de pavot, les protéines de lupin, les protéines de tournesol, les protéines d'avoine, les protéines de millet, les protéines d'orge, les protéines de seigle, leurs dérivés et leurs mélanges, des saccharides naturels ou synthétiques sélectionnés parmi l'alginate de calcium, l'alginate de sodium, l'alginate d'ammonium, l'alginate de propylène glycol, les amidons, les amidons prégélatinisés, les amidons modifiés, les fibres solubles ou insolubles, les carraghénanes, la pectine, l'hydroxypropylcellulose (HPC), le glycogène, le β-glucane, l'amylopectine, l'amylose, les dextrines, les cyclodextrines, la maltodextrine, l'isomaltose, l'isomalt, le xylane, le pullulan, l'agar-agar, les mannanes, le fucoïdane, la gomme de xanthane, la gomme de guar, la gomme mastic, la gomme arabique, la gomme d'acacia, la gomme adragante, la gomme ghatti, la gomme de karaya, la gomme de Konjac, la gomme de fenugrec, la gomme d'acajou ou anacarde, la gomme tara, la gomme de caroube, la gomme de cassia, la gomme de benjoin, la gomme cassia, la gomme gaïac, le chitosan, la chitine, la lévane, la néoserine, l'acide hyaluronique, les hyaluronates, le sulfate de chondroïtine, le dermatane sulfate, le kératane sulfate, le raffinose, le rhamminose, le mannose, le glucose, le rhamnose, le saccharose, le stachyose, le verbascose, le tréhalose, le lactose, le lactulose, le maltose, l'érythritol, le mannitol, le sorbitol, le xylitol, leurs dérivés et leurs mélanges, des lipides naturels ou synthétiques sélectionnés parmi les cires d'abeille, la cire de candelilla, la cire de son de riz, la cire de carnauba, les cires microcristallines, les huiles animales ou végétales, les huiles animales ou végétales hydrogénées, les phospholipides, les céramides, les esters d'acide gras, leurs dérivés et leurs mélanges, de l'urée, de ses dérivés et leurs mélanges ;
b) une étape d'amenée dudit pré-mélange formé à l'étape a) pour alimenter un extrudeur de type Process 11 Hygienic de Thermo-Fischer^{®} ;
c) une étape de mélange, dans ledit extrudeur, dudit pré-mélange pour obtenir un mélange ;
d) une étape de thermoformage par extrusion à chaud dudit mélange obtenu à l'étape c) dans ledit extrudeur pour obtenir un extrudat thermoformé friable / cassant, l'étape d'extrusion à chaud étant réalisée à une vitesse de rotation d'une vis d'extrusion de 200 tours/minute et à une température comprise entre 20 et 80°C ;
e) une étape de refroidissement en sortie de l'extrudeur dudit extrudat thermoformé friable / cassant obtenu à l'étape d) ; et
f) une étape de découpe/broyage, au niveau d'un broyeur, de l'extrudat thermoformé friable / cassant refroidi obtenu à l'étape e) de telle sorte à obtenir une poudre homogène, c'est-à-dire un extrudat thermoformé conditionné en poudre (sous forme de poudre).

La température de thermoformage à laquelle est réalisée l'étape d'extrusion à chaud est déterminée par le type de constituants mis en œuvre, en particulier selon le type de support et/ou d'agent plastifiant mis en œuvre, ce que l'homme de métier est à même de déterminer. Par ailleurs, un homme de métier, notamment selon le type d'extrudeur employé et conformément au principe général de l'extrusion à chaud (HME), est à même de définir d'éventuels paliers de températures en différentes zones le long de la ou des vis d'extrusion de telle sorte qu'ait lieu une augmentation progressive de la température au sein de la matière transportée par la ou des vis d'extrusion, ceci dans un sens d'avancement de la matière au sein de l'extrudeur. Typiquement, entre zones définies le long de la ou des vis d'extrusion, des différences de températures de l'ordre de 0 à 40°C sont observées. Par exemple, dans le cadre de la présente invention, les compositions testées ci-après ont été obtenues dans un extrudeur de type Process 11 Hygienic de Thermo-Fischer^{®} présentant 8 zones de températures qui sont les suivantes dans un sens d'avancée de la matière évoluant à une vitesse de 200 tours/minute : 20°C - 40°C - 60°C - 60°C - 80°C - 80°C - 80°C et 80°C.

### Exemple 2 : test de dispersion et de solubilité de compositions thermoformées selon l'invention

Des compositions thermoformées, obtenues selon le procédé de fabrication décrit à l'exemple 1, ont été testées en termes de dispersion et de solubilité du cannabidiol. Cette dispersion et cette solubilité ont été mesurées au cours du temps au départ d'extrudats thermoformés obtenus selon l'invention. Comme indiqué ci-dessus, les extrudats thermoformés se présentent sous la forme d'une poudre homogène (broyât) dans laquelle le cannabidiol (un cannabinoïde non-euphorisant) comprend préférentiellement au moins une phase amorphe.

Le test de dispersion et de solubilité a été réalisé avec un appareil à dissolution à palettes au départ d'environ 360 mg d'extrudat thermoformé, à une température de 37°C sous agitation à 50 tours/minute dans 900 ml d'un milieu de dissolution HCI 0,1N. Ce test de dispersion et de solubilité a été mené selon les recommandations de la pharmacopée Ph.Eur.9.0 (Recommendations on Dissolution Testing). A des temps déterminés (après 15 min, après 30 min, après 1h et après 2h), un échantillon de 1 ml est prélevé pour réaliser le test de dispersion et de solubilité.

Afin de réaliser le test de dispersion et de solubilité, l'échantillon est dilué dans un solvant approprié (méthanol) puis filtré au travers d'un filtre (PET, taille des pores de 0,45 µm, Macherey Nagel) avant analyse HPLC (colonne Nucleodur 100-5 EC C18 125/4 (Macherey-Nagel) ; phase mobile : 85% -A (Méthanol) et 15% B (Eau) ; débit : 1 ml/min ; loop : 10 µl, t° = 25°C ; 220 nm).

Les compositions thermoformées selon l'invention reprises au Tableau 1 ont été formulées suivant le procédé de l'invention et testées en termes de dispersion et de solubilité au cours du temps selon le principe indiqué ci-dessus. Du cannabidiol (CBD) sous forme native cristalline et sous forme de poudre (CBD natif) a été utilisée en tant que contrôle. Les quantités mentionnées au Tableau 1 sont des pourcentages en poids des composés mis en œuvre (soumis au procédé selon l'invention) par rapport au poids total de la composition.

**Tableau 1**

| | Cannabidiol (CBD) (1) | Protéine (2) | Saccharide (3) | Glycérol (4) |
|---|---|---|---|---|
| Compo 1 | 20 | 70 | 0 | 10 |
| Compo 2 | 20 | 0 | 70 | 10 |

| | | | | |
|---|---|---|---|---|
| (1) Cannabidiol (Shanghai Freemen) (2) Collagène bovin 5000 Da (Rousselot) (3) Amidon modifié Cleargum^{®} CO01 (Roquette) (4) Glycérol (Brenntag) | | | | |

Les résultats des tests de dispersion et de solubilité obtenus sont présentés à la figure 1. Comme on peut le constater, la dispersion + la solubilité mesurées pour le CBD au départ de compositions sous forme d'extrudats thermoformés suivant l'invention (Compo 1 et Compo 2) est nettement supérieure par rapport au contrôle (CBD natif).

### Exemple 3: test de dispersion et de solubilité de compositions thermoformées selon l'invention

Des compositions thermoformées, obtenues selon le procédé de fabrication décrit à l'exemple 1, ont été testées en termes de dispersion et de solubilité du cannabidiol. Cette dispersion et cette solubilité ont été mesurées au cours du temps au départ d'extrudats thermoformés obtenus selon l'invention. Comme indiqué ci-dessus, les extrudats thermoformés se présentent sous la forme d'une poudre homogène (broyât) dans laquelle le cannabidiol (un cannabinoïde non-euphorisant) comprend préférentiellement au moins une phase amorphe.

Le test de dispersion et de solubilité a été réalisé comme indiqué à l'exemple 2.

Les compositions thermoformées selon l'invention reprises au Tableau 2 ont été formulées suivant le procédé de l'invention et testées en termes de dispersion et de solubilité au cours du temps selon le principe indiqué plus haut. Du cannabidiol (CBD) sous forme native cristalline et sous forme de poudre (CBD natif) a été utilisée en tant que contrôle. Les quantités mentionnées au Tableau 2 sont des pourcentages en poids des composés mis en œuvre (soumis au procédé selon l'invention) par rapport au poids total de la composition.

**Tableau 2**

| | Cannabidiol (CBD) (1) | Protéine (2) | Glycérol (3) |
|---|---|---|---|
| Compo 1 | 20 | 70 | 10 |
| Compo 2 | 25 | 70 | 5 |
| Compo 3 | 30 | 70 | 0 |

| | | | |
|---|---|---|---|
| (1) Cannabidiol (Shanghai Freemen) (2) Collagène Peptan B 5000 HD (Ingrizo) (3) Glycérol (Brenntag) | | | |

Les résultats des tests de dispersion et de solubilité obtenus sont présentés à la figure 2. Comme on peut le constater, la dispersion + la solubilité mesurées pour le CBD au départ de compositions sous forme d'extrudats thermoformés suivant l'invention (Compo 1, Compo 2 et Compo 3) est nettement supérieure par rapport au contrôle (CBD natif).

### Exemple 4: test de dispersion et de solubilité de compositions thermoformées selon l'invention

Des compositions thermoformées, obtenues selon le procédé de fabrication décrit à l'exemple 1, ont été testées en termes de dispersion et de solubilité du cannabidiol. Cette dispersion et cette solubilité ont été mesurées au cours du temps au départ d'extrudats thermoformés obtenus selon l'invention. Comme indiqué ci-dessus, les extrudats thermoformés se présentent sous la forme d'une poudre homogène (broyât) dans laquelle le cannabidiol (un cannabinoïde non-euphorisant) comprend préférentiellement au moins une phase amorphe.

Le test de dispersion et de solubilité a été réalisé comme indiqué à l'exemple 2.

Les compositions thermoformées selon l'invention reprises au Tableau 3 ont été formulées suivant le procédé de l'invention et testées en termes de dispersion et de solubilité au cours du temps selon le principe indiqué plus haut. Du cannabidiol (CBD) sous forme native cristalline et sous forme de poudre (CBD natif) a été utilisée en tant que contrôle. Les quantités mentionnées au Tableau 3 sont des pourcentages en poids des composés mis en œuvre (soumis au procédé selon l'invention) par rapport au poids total de la composition.

**Tableau 3**

| | Cannabidiol (CBD) (1) | Saccharide | Glycérol (6) |
|---|---|---|---|
| Compo 1 | 20 | 70 (2) | 10 |
| Compo 2 | 20 | 70 (3) | 10 |
| Compo 3 | 20 | 70 (4) | 10 |
| Compo 4 | 20 | 70 (5) | 10 |

| | | | |
|---|---|---|---|
| (1) Cannabidiol (Shanghai Freemen) (2) Dextrine Tackidex^{®} C760 (Roquette) (3) Amidon modifié Cleargum^{®} CB95 (Roquette) (4) Amidon modifié Cleargum^{®} MG85 (Roquette) (5) Amidon modifié Cleargum^{®} LG7015 (Roquette) (6) Glycérol (Brenntag) | | | |

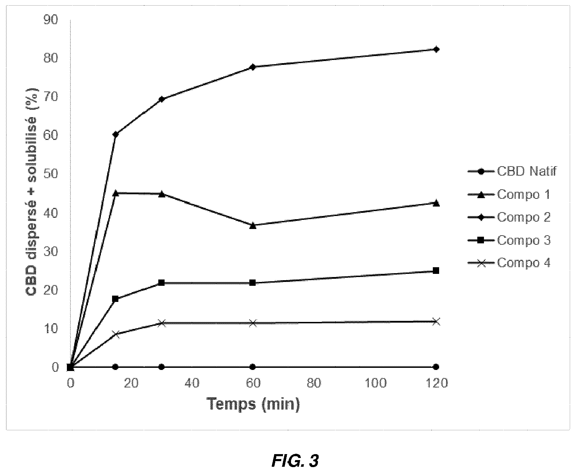

Les résultats des tests de dispersion et de solubilité obtenus sont présentés à la figure 3. Comme on peut le constater, la dispersion + la solubilité mesurées pour le CBD au départ de compositions sous forme d'extrudats thermoformés suivant l'invention (Compo 1, Compo 2, Compo 3 et Compo 4) est toujours supérieure par rapport au contrôle (CBD natif).

La présente invention a été décrite en relation avec des modes de réalisations spécifiques, qui ont une valeur purement illustrative et ne doivent pas être considérés comme limitatifs. D'une manière générale, il apparaîtra évident pour l'homme du métier que la présente invention n'est pas limitée aux exemples illustrés et/ou décrits ci-dessus.

L'usage des verbes « comprendre », « inclure », « comporter », ou toute autre variante, ainsi que leurs conjugaisons, ne peut en aucune façon exclure la présence d'éléments autres que ceux mentionnés.

L'usage de l'article indéfini « un », « une », ou de l'article défini « le », « la » ou « l' », pour introduire un élément n'exclut pas la présence d'une pluralité de ces éléments.

## Revendications

1. Composition sous forme d'un extrudat thermoformé friable / cassant, éventuellement conditionné en pellet, en flake, en granulé, en poudre, en comprimé effervescent ou non, en solution injectable ou non, en suspension, en gel, en pommade ou encore sous toute autre forme adéquate permettant une administration à un animal ou à un être humain, comprenant au moins un cannabinoïde non-euphorisant naturel ou synthétique et au moins un support naturel ou synthétique choisi dans le groupe constitué des acides aminés, des peptides et des polypeptides naturels ou synthétiques, des protéines naturelles ou synthétiques sélectionnées parmi les glycoprotéines, les collagènes, les hydrolysats de collagènes, les gélatines, les protéines de pois, les protéines de soja, les protéines de blé, les protéines de citrouille, les protéines de noix, les protéines de riz, les protéines de noisette, les protéines de lait, les protéines de petit pois, les protéines de lentilles, les protéines d'amandes, les protéines de pistaches, les protéines de chia, les protéines de chanvre, les protéines de lin, les protéines de courge, les protéines de sésame, les protéines de pavot, les protéines de lupin, les protéines de tournesol, les protéines d'avoine, les protéines de millet, les protéines d'orge, les protéines de seigle, leurs dérivés et leurs mélanges, des saccharides naturels ou synthétiques sélectionnés parmi l'alginate de calcium, l'alginate de sodium, l'alginate d'ammonium, l'alginate de propylène glycol,les amidons, les amidons prégélatinisés, les amidons modifiés, les fibres solubles ou insolubles, les carraghénanes, la pectine, l'hydroxypropylcellulose (HPC), le glycogène, le β-glucane, l'amylopectine, l'amylose, les dextrines, les cyclodextrines, la maltodextrine, l'isomaltose, l'isomalt, le xylane, le pullulan, l'agar-agar, les mannanes, le fucoïdane, la gomme de xanthane, la gomme de guar, la gomme mastic, la gomme arabique, la gomme d'acacia, la gomme adragante, la gomme ghatti, la gomme de karaya, la gomme de Konjac, la gomme de fenugrec, la gomme d'acajou ou anacarde, la gomme tara, la gomme de caroube, la gomme de cassia, la gomme de benjoin, la gomme cassia, la gomme gaïac, le chitosan, la chitine, la lévane, la néoserine, l'acide hyaluronique, les hyaluronates, le sulfate de chondroïtine, le dermatane sulfate, le kératane sulfate, le raffinose, le rhamminose, le mannose, le glucose, le rhamnose, le saccharose, le stachyose, le verbascose, le tréhalose, le lactose, le lactulose, le maltose, l'érythritol, le mannitol, le sorbitol, le xylitol, leurs dérivés et leurs mélanges, des lipides naturels ou synthétiques sélectionnés parmi les cires d'abeille, la cire de candelilla, la cire de son de riz, la cire de carnauba, les cires microcristallines, les huiles animales ou végétales, les huiles animales ou végétales hydrogénées, les phospholipides, les céramides, les esters d'acide gras, leurs dérivés et leurs mélanges, de l'urée, de ses dérivés et leurs mélanges.

2. Composition selon la revendication 1, **caractérisée en ce que** ledit extrudat thermoformé friable / cassant comprend un mélange thermoformé d'au moins un cannabinoïde non-euphorisant naturel ou synthétique et d'au moins un support naturel ou synthétique choisi dans le groupe constitué des acides aminés, des peptides et des polypeptides naturels ou synthétiques, des protéines naturelles ou synthétiques sélectionnées parmi les glycoprotéines, les collagènes, les hydrolysats de collagènes, les gélatines, les protéines de pois, les protéines de soja, les protéines de blé, les protéines de citrouille, les protéines de noix, les protéines de riz, les protéines de noisette, les protéines de lait, les protéines de petit pois, les protéines de lentilles, les protéines d'amandes, les protéines de pistaches, les protéines de chia, les protéines de chanvre, les protéines de lin, les protéines de courge, les protéines de sésame, les protéines de pavot, les protéines de lupin, les protéines de tournesol, les protéines d'avoine, les protéines de millet, les protéines d'orge, les protéines de seigle, leurs dérivés et leurs mélanges, des saccharides naturels ou synthétiques sélectionnés parmi l'alginate de calcium, l'alginate de sodium, l'alginate d'ammonium, l'alginate de propylène glycol, les amidons, les amidons prégélatinisés, les amidons modifiés, les fibres solubles ou insolubles, les carraghénanes, la pectine, l'hydroxypropylcellulose (HPC), le glycogène, le β-glucane, l'amylopectine, l'amylose, les dextrines, les cyclodextrines, la maltodextrine, l'isomaltose, l'isomalt, le xylane, le pullulan, l'agar-agar, les mannanes, le fucoïdane, la gomme de xanthane, la gomme de guar, la gomme mastic, la gomme arabique, la gomme d'acacia, la gomme adragante, la gomme ghatti, la gomme de karaya, la gomme de Konjac, la gomme de fenugrec, la gomme d'acajou ou anacarde, la gomme tara, la gomme de caroube, la gomme de cassia, la gomme de benjoin, la gomme cassia, la gomme gaïac, le chitosan, la chitine, la lévane, la néoserine, l'acide hyaluronique, les hyaluronates, le sulfate de chondroïtine, le dermatane sulfate, le kératane sulfate, le raffinose, le rhamminose, le mannose, le glucose, le rhamnose, le saccharose, le stachyose, le verbascose, le tréhalose, le lactose, le lactulose, le maltose, l'érythritol, le mannitol, le sorbitol, le xylitol, leurs dérivés et leurs mélanges, des lipides naturels ou synthétiques sélectionnés parmi les cires d'abeille, la cire de candelilla, la cire de son de riz, la cire de carnauba, les cires microcristallines, les huiles animales ou végétales, les huiles animales ou végétales hydrogénées, les phospholipides, les céramides, les esters d'acide gras, leurs dérivés et leurs mélanges, de l'urée, de ses dérivés et leurs mélanges.

3. Composition selon l'une quelconque des revendications précédentes, **caractérisée en ce que** ledit au moins un cannabinoïde non-euphorisant naturel ou synthétique est choisi dans le groupe constitué du du cannabidiol (CBD), du cannabigérol (CBG), du cannabichromène (CBC), de la cannabidivarine (CBDV), de l'acide cannabidiolique (CBDA), de la cannabigérovarine (CBGV), de l'acide cannabichroménique (CBCA), de l'acide cannabiorcichroménique, de l'acide 8-chloro-cannabiorcichroménique, de l'acide cannachromévarinique (CBCVA), de la cannabielsoïne (CBE), du cannabicyclol (CBL), du perrottetinene, de l'acide perrottittenique, de l'acide lunularique, de la vitatine, de l'helichrysum CBG, des amorfrutines (A, B, C et D), des machaeridiols (A, B, C), de l'acide daurichroménique, de l'acide anthopogocyclolique, de l'acide anthopogochroménique, de l'acide geranyl-orsellinique, de la confluentine, de l'acide griffolique, du rubiginosin A, de leurs dérivés et de leurs mélanges.

4. Composition selon l'une quelconque des revendications précédentes, **caractérisée en ce que** lesdits collagènes et/ou lesdits hydrolysats de collagène et/ou lesdites gélatines présentent un poids moléculaire compris entre 50 et 300000 Da, de préférence entre 100 et 275000 Da, de préférence entre 150 et 250000 Da, de préférence entre 200 et 225000 Da, de préférence entre 250 et 200000 Da, de préférence entre 300 et 175000 Da, de préférence entre 350 et 150000 Da, de préférence entre 400 et 125000 Da, de préférence entre 450 et 100000 Da, de préférence entre 500 et 75000 Da, de préférence entre 550 et 50000 Da, de préférence entre 600 et 40000 Da, de préférence entre 650 et 30000 Da, de préférence entre 700 et 20000 Da, de préférence entre 750 et 10000 Da, de préférence entre 800 et 9000 Da, de préférence entre 850 et 8000 Da, de préférence entre 900 et 7000 Da, de préférence entre 950 et 6000 Da, de préférence entre 1000 et 5000 Da, de préférence entre 1050 et 4000 Da, de préférence entre 1100 et 3000 Da, de préférence entre 1150 et 2000 Da, de préférence entre 1200 et 1000 Da.

5. Composition selon l'une quelconque des revendications précédentes, **caractérisée en ce qu'**elle comprend en outre au moins un agent plastifiant.

6. Composition selon l'une quelconque des revendications précédentes, **caractérisée en ce qu'**elle comprend en outre au moins un additif choisi dans le groupe constitué des agents lubrifiants, des agents surfactants, des agents antioxydants, des agents chélatants, de leurs dérivés et de leurs mélanges.

7. Composition selon l'une quelconque des revendications précédentes, **caractérisée en ce qu'**elle comprend en outre au moins un premier composé additionnel de type polyphénol choisi dans le groupe constitué des acides phénoliques, des stilbènes, des alcools phénoliques, des lignanes, des flavonoïdes, de leurs dérivés et de leurs mélanges.

8. Procédé de fabrication, en particulier procédé de fabrication par thermoformage, d'une composition sous forme d'un extrudat thermoformé friable / cassant selon l'une quelconque des revendications 1 à 7, **caractérisé en ce qu'**il comprend les étapes suivantes :
a) une étape d'amenée simultanée ou différée dans le temps d'au moins un cannabinoïde non-euphorisant naturel ou synthétique et d'au moins un support naturel ou synthétique choisi dans le groupe constitué des acides aminés, des peptides et des polypeptides naturels ou synthétiques, des protéines naturelles ou synthétiques sélectionnées parmi les glycoprotéines, les collagènes, les hydrolysats de collagènes, les gélatines, les protéines de pois, les protéines de soja, les protéines de blé, les protéines de citrouille, les protéines de noix, les protéines de riz, les protéines de noisette, les protéines de lait, les protéines de petit pois, les protéines de lentilles, les protéines d'amandes, les protéines de pistaches, les protéines de chia, les protéines de chanvre, les protéines de lin, les protéines de courge, les protéines de sésame, les protéines de pavot, les protéines de lupin, les protéines de tournesol, les protéines d'avoine, les protéines de millet, les protéines d'orge, les protéines de seigle, leurs dérivés et leurs mélanges, des saccharides naturels ou synthétiques sélectionnés parmi l'alginate de calcium, l'alginate de sodium, l'alginate d'ammonium, l'alginate de propylène glycol, les amidons, les amidons prégélatinisés, les amidons modifiés, les fibres solubles ou insolubles, les carraghénanes, la pectine, l'hydroxypropylcellulose (HPC), le glycogène, le β-glucane, l'amylopectine, l'amylose, les dextrines, les cyclodextrines, la maltodextrine, l'isomaltose, l'isomalt, le xylane, le pullulan, l'agar-agar, les mannanes, le fucoïdane, la gomme de xanthane, la gomme de guar, la gomme mastic, la gomme arabique, la gomme d'acacia, la gomme adragante, la gomme ghatti, la gomme de karaya, la gomme de Konjac, la gomme de fenugrec, la gomme d'acajou ou anacarde, la gomme tara, la gomme de caroube, la gomme de cassia, la gomme de benjoin, la gomme cassia, la gomme gaïac, le chitosan, la chitine, la lévane, la néoserine, l'acide hyaluronique, les hyaluronates, le sulfate de chondroïtine, le dermatane sulfate, le kératane sulfate, le raffinose, le rhamminose, le mannose, le glucose, le rhamnose, le saccharose, le stachyose, le verbascose, le tréhalose, le lactose, le lactulose, le maltose, l'érythritol, le mannitol, le sorbitol, le xylitol, leurs dérivés et leurs mélanges, des lipides naturels ou synthétiques sélectionnés parmi les cires d'abeille, la cire de candelilla, la cire de son de riz, la cire de carnauba, les cires microcristallines, les huiles animales ou végétales, les huiles animales ou végétales hydrogénées, les phospholipides, les céramides, les esters d'acide gras, leurs dérivés et leurs mélanges, de l'urée, de ses dérivés et leurs mélanges, pour alimenter un extrudeur,
b) une étape de mélange, dans ledit extrudeur, dudit au moins un cannabinoïde non-euphorisant naturel ou synthétique et dudit au moins un support naturel ou synthétique choisi dans le groupe constitué des acides aminés, des peptides et des polypeptides naturels ou synthétiques, des protéines naturelles ou synthétiques sélectionnées parmi les glycoprotéines, les collagènes, les hydrolysats de collagènes, les gélatines, les protéines de pois, les protéines de soja, les protéines de blé, les protéines de citrouille, les protéines de noix, les protéines de riz, les protéines de noisette, les protéines de lait, les protéines de petit pois, les protéines de lentilles, les protéines d'amandes, les protéines de pistaches, les protéines de chia, les protéines de chanvre, les protéines de lin, les protéines de courge, les protéines de sésame, les protéines de pavot, les protéines de lupin, les protéines de tournesol, les protéines d'avoine, les protéines de millet, les protéines d'orge, les protéines de seigle, leurs dérivés et leurs mélanges, des saccharides naturels ou synthétiques sélectionnés parmi l'alginate de calcium, l'alginate de sodium, l'alginate d'ammonium, l'alginate de propylène glycol, les amidons, les amidons prégélatinisés, les amidons modifiés, les fibres solubles ou insolubles, les carraghénanes, la pectine, l'hydroxypropylcellulose (HPC), le glycogène, le β-glucane, l'amylopectine, l'amylose, les dextrines, les cyclodextrines, la maltodextrine, l'isomaltose, l'isomalt, le xylane, le pullulan, l'agar-agar, les mannanes, le fucoïdane, la gomme de xanthane, la gomme de guar, la gomme mastic, la gomme arabique, la gomme d'acacia, la gomme adragante, la gomme ghatti, la gomme de karaya, la gomme de Konjac, la gomme de fenugrec, la gomme d'acajou ou anacarde, la gomme tara, la gomme de caroube, la gomme de cassia, la gomme de benjoin, la gomme cassia, la gomme gaïac, le chitosan, la chitine, la lévane, la néoserine, l'acide hyaluronique, les hyaluronates, le sulfate de chondroïtine, le dermatane sulfate, le kératane sulfate, le raffinose, le rhamminose, le mannose, le glucose, le rhamnose, le saccharose, le stachyose, le verbascose, le tréhalose, le lactose, le lactulose, le maltose, l'érythritol, le mannitol, le sorbitol, le xylitol, leurs dérivés et leurs mélanges, des lipides naturels ou synthétiques sélectionnés parmi les cires d'abeille, la cire de candelilla, la cire de son de riz, la cire de carnauba, les cires microcristallines, les huiles animales ou végétales, les huiles animales ou végétales hydrogénées, les phospholipides, les céramides, les esters d'acide gras, leurs dérivés et leurs mélanges, de l'urée, de ses dérivés et leurs mélanges, pour former un mélange, et
c) une étape d'extrusion à chaud dudit mélange obtenu à l'étape b) dans ledit extrudeur pour obtenir un extrudat thermoformé friable / cassant, en particulier pour obtenir un extrudat thermoformé friable / cassant consistant en une dispersion solide, plus particulièrement pour obtenir un extrudat thermoformé consistant en une dispersion solide consistant en une structure vitreuse comprenant un mélange moléculaire dudit au moins un cannabinoïde non-euphorisant et dudit au moins un support.

9. Procédé selon la revendication 8, **caractérisé en ce qu'**il comprend une étape préalable de pré-mélange dudit au moins un cannabinoïde non-euphorisant naturel ou synthétique et dudit au moins un support naturel ou synthétique choisi dans le groupe constitué des acides aminés, des peptides et des polypeptides naturels ou synthétiques, des protéines naturelles ou synthétiques sélectionnées parmi les glycoprotéines, les collagènes, les hydrolysats de collagènes, les gélatines, les protéines de pois, les protéines de soja, les protéines de blé, les protéines de citrouille, les protéines de noix, les protéines de riz, les protéines de noisette, les protéines de lait, les protéines de petit pois, les protéines de lentilles, les protéines d'amandes, les protéines de pistaches, les protéines de chia, les protéines de chanvre, les protéines de lin, les protéines de courge, les protéines de sésame, les protéines de pavot, les protéines de lupin, les protéines de tournesol, les protéines d'avoine, les protéines de millet, les protéines d'orge, les protéines de seigle, leurs dérivés et leurs mélanges, des saccharides naturels ou synthétiques sélectionnés parmi l'alginate de calcium, l'alginate de sodium, l'alginate d'ammonium, l'alginate de propylène glycol, les amidons, les amidons prégélatinisés, les amidons modifiés, les fibres solubles ou insolubles, les carraghénanes, la pectine, l'hydroxypropylcellulose (HPC), le glycogène, le β-glucane, l'amylopectine, l'amylose, les dextrines, les cyclodextrines, la maltodextrine, l'isomaltose, l'isomalt, le xylane, le pullulan, l'agar-agar, les mannanes, le fucoïdane, la gomme de xanthane, la gomme de guar, la gomme mastic, la gomme arabique, la gomme d'acacia, la gomme adragante, la gomme ghatti, la gomme de karaya, la gomme de Konjac, la gomme de fenugrec, la gomme d'acajou ou anacarde, la gomme tara, la gomme de caroube, la gomme de cassia, la gomme de benjoin, la gomme cassia, la gomme gaïac, le chitosan, la chitine, la lévane, la néoserine, l'acide hyaluronique, les hyaluronates, le sulfate de chondroïtine, le dermatane sulfate, le kératane sulfate, le raffinose, le rhamminose, le mannose, le glucose, le rhamnose, le saccharose, le stachyose, le verbascose, le tréhalose, le lactose, le lactulose, le maltose, l'érythritol, le mannitol, le sorbitol, le xylitol, leurs dérivés et leurs mélanges, des lipides naturels ou synthétiques sélectionnés parmi les cires d'abeille, la cire de candelilla, la cire de son de riz, la cire de carnauba, les cires microcristallines, les huiles animales ou végétales, les huiles animales ou végétales hydrogénées, les phospholipides, les céramides, les esters d'acide gras, leurs dérivés et leurs mélanges, de l'urée, de ses dérivés et leurs mélanges, de telle sorte à former un pré-mélange destiné à alimenter l'extrudeur.

10. Procédé selon la revendication 8 ou 9, **caractérisé en ce que** ladite étape d'extrusion à chaud est réalisée à une température d'extrusion comprise entre 20 et 180°C, de préférence à une température comprise entre 40 et 115°C, préférentiellement à une température comprise entre 80 et 90°C, de préférence à une température égale à 115°C, de préférence à une température égale à 110°C, de préférence à une température égale à 100°C, de préférence à une température égale à 90°C, de préférence à une température égale à 80°C.

11. Procédé selon l'une quelconque des revendications 8 à 10, **caractérisé en ce que** ladite étape d'extrusion à chaud est réalisée à une vitesse de rotation d'une vis d'extrusion comprise entre 20 et 900 tours/min, de préférence comprise entre 50 et 300 tours/min, de préférence comprise entre 100 et 250 tours/min, préférentiellement égale à 250 tours/min, préférentiellement égale à 200 tours/min.

12. Procédé selon l'une quelconque des revendications 8 à 11, **caractérisé en ce qu'**il comprend une étape additionnelle de refroidissement en sortie de l'extrudeur.

13. Procédé selon l'une quelconque des revendications 8 à 12, **caractérisé en ce qu'**il comprend une étape additionnelle de traitement de l'extrudat thermoformé friable / cassant en sortie de l'extrudeur, par exemple une découpe au niveau d'un pelletiseur et/ou un broyage et/ou un concassage et/ou une réduction en poudre dudit extrudat thermoformé.

14. Composition sous forme d'un extrudat thermoformé friable / cassant, éventuellement conditionné en pellet, en flake, en granulé, en poudre, en comprimé effervescent ou non, en solution injectable ou non, en suspension, en gel, en pommade ou encore sous toute autre forme adéquate permettant une administration à un animal ou à un être humain, selon l'une quelconque des revendications 1 à 7 pour utilisation dans le traitement préventif et/ou curatif, chez l'être humain et/ou chez l'animal, de pathologies en lien avec le système cardiovasculaire (ischémie, désordres métaboliques,...), de pathologies en lien avec le système gastro-intestinal (nausées, vomissements, diarrhées, inflammations digestives, ...), de pathologies en lien avec le vieillissement prématuré des cellules, de pathologies en lien avec le système endocrinien (hyperglycémie, ...), de pathologies en lien avec le système immunitaire (maladies inflammatoires, arthrite rhumatoïde, sclérose en plaque, ostéoarthrite, fibromyalgie,...), de pathologies en lien avec le système nerveux central (douleur, migraine, épilepsie, maladie de Parkinson, maladie d'Alzheimer, anxiété, dépression,...), des troubles du sommeil, de maladies de la peau, de maladies dues à la présence de microorganismes, des cancers (anti-tumoral, ...), des maladies oculaires (glaucoma,...), des maladies du système respiratoire (asthme,...) et dans le traitement préventif et/ou curatif du diabète.

15. Composition sous forme d'un extrudat thermoformé friable / cassant, éventuellement conditionné en pellet, en flake, en granulé, en poudre, en comprimé effervescent ou non, en solution injectable ou non, en suspension, en gel, en pommade ou encore sous toute autre forme adéquate permettant une administration à un animal ou à un être humain, obtenu suivant le procédé selon l'une quelconque des revendications 8 à 13.
